# EUROPEAN PATENT SPECIFICATION

(11) **EP 2 914 198 B1**
(45) Date of publication and mention of the grant of the patent: **17.06.2020**
(21) Application number: 13851407.0
(22) Date of filing: 01.11.2013
(51) Int. Cl.: A61B 34/30, A61B 18/14, A61B 90/98, A61B 17/00, A61B 18/00

(54) **FLUX TRANSMISSION CONNECTORS AND SYSTEMS, FLUX DISAMBIGUATION, AND SYSTEMS AND METHODS FOR MAPPING FLUX SUPPLY PATHS**
FLUSSÜBERTRAGUNGSVERBINDER UND -SYSTEME, FLUSSUNTERSCHEIDUNG SOWIE SYSTEME UND VERFAHREN ZUR KARTIERUNG VON FLUSSZUFUHRPFADEN
RACCORDS ET SYSTÈMES DE TRANSMISSION DE FLUX, DÉSAMBIGUÏSATION DE FLUX, ET SYSTÈMES ET PROCÉDÉS DE MAPPAGE DE TRAJETS D'ALIMENTATION EN FLUX

(30) Priority: 02.11.2012 US 201261721863 P; 02.11.2012 US 201261721870 P; 02.11.2012 US 201261721909 P
(43) Date of publication of application: 09.09.2015
(60) Divisional application: 20172390.5
(73) Proprietor: Intuitive Surgical Operations, Inc., Sunnyvale, CA 94086 (US)
(72) Inventor: DACHS, Gregory W., Sunnyvale, California 94086 (US); MILLMAN, Paul, Sunnyvale, California 94086 (US); MOHR, Paul W., Sunnyvale, California 94086 (US); SCHENA, Bruce M., Sunnyvale, California 94086 (US); HOLOP, Robert E., Sunnyvale, California 94086 (US); COOPER, Thomas G., Sunnyvale, California 94086 (US)
(74) Representative: MacDougall, Alan John Shaw
(86) International application number: PCT/US2013/068059
(87) International publication number: WO 2014/071184

(56) References cited:
- WO-A1-2010/008126
- US-A1- 2008 020 714
- US-A1- 2009 024 142
- US-A1- 2009 275 940
- US-A1- 2010 228 264
- US-A1- 2010 305 427
- US-A1- 2011 045 680
- US-A1- 2012 202 388
- US-B1- 6 237 604

## Description

### TECHNICAL FIELD

Aspects of the present disclosure relate to systems and methods for automated detection and mapping of flux supply paths between multiple flux supply sources and multiple remotely-controllable flux delivery structures. More particularly, aspects of the present disclosure relate to systems and methods for determining which of a plurality of electrosurgical instruments installed at patient side cart of a robotic (teleoperated) surgical system is in energy communication with which of a plurality of electrical energy supply sources, and controlling energy supply based on the same. Aspects of the present disclosure further relate to surgical instruments for teleoperated surgical systems, and related systems, devices and methods for controlling delivery of a flux supplied from an external source to such instruments.

### INTRODUCTION

Some minimally invasive surgical techniques are performed remotely through the use of robotically-controlled (teleoperated) surgical instruments. In robotically-controlled (teleoperated) surgical systems, surgeons manipulate input devices at a surgeon console, and those inputs are passed to a patient side cart that interfaces with one or more teleoperated surgical instruments. Based on the surgeon's inputs at the surgeon console, the one or more teleoperated surgical instruments are actuated at the patient side cart to operate on the patient, thereby creating a master-slave control relationship between the surgeon console and the surgical instrument(s) at the patient side cart.

Some surgical instruments are configured for delivery of a flux to a patient. Such instruments can be placed in operational connection with a flux source that generates and supplies flux to the surgical instrument to be delivered to a patient during a surgical procedure.

By way of particular example, electrosurgical instruments can be placed in communication with an electrical energy generator to deliver cautery energy in teleoperated surgical systems to perform a cautery procedure (e.g., tissue ablation, tissue sealing, tissue cutting, etc.). The surgical instruments that delivery cautery energy may include, for example, monopolar instruments or bipolar instruments. Monopolar instruments typically deliver electrical energy through a single source electrode and a return, or sink, electrode that returns electrical energy back to an energy generator disposed externally to the patient. Examples of monopolar electrosurgical instruments include, but are not limited to, hooks, spatulas, shears including two blades energized with the same electric potential, cautery probes, irrigators, scissors, etc. Bipolar instruments typically deliver electrical energy through two electrodes (e.g., source and sink electrodes), typically two jaws of the surgical instrument, separately, and the return path for the current is from one pole through the other pole. Examples of bipolar instruments include, but are not limited to, graspers, forceps, clamps, etc., which are generally used for sealing vessels and vascular tissue, grasping vessels, cauterizing or coagulating tissue, etc. Other types of energy (e.g., ultrasound and/or laser) also may be delivered to the patient through surgical instruments mounted at the patient side cart.

Electrosurgical instruments, and others that deliver flux to the patient, are somewhat unique in that, in addition to being coupled to various actuation interface mechanisms at the patient side cart to control movement of the instrument based on the master inputs, they also are in communication with a flux source, e.g., an electrical energy generator in communication with an electrosurgical instrument. As with the movement of the instrument in general, flux delivery from such a surgical instrument to the patient is responsive to an input (e.g., pressing of a foot pedal or other input device) at the surgeon console.

It may be desirable for various reasons to have more than one surgical instrument configured for flux delivery (e.g., more than one electrosurgical instrument) mounted at the patient side cart during a teleoperated surgical procedure. A need exists, however, to provide a teleoperated teleoperated surgical system that can reliably and in an automated manner determine which one(s) of a plurality of surgical instruments mounted at a patient side cart is operationally coupled with a specific flux supply source. There also exists a need to provide various control schemes and automated control methods relating to flux delivery to surgical instruments of teleoperated surgical systems. Further, there exists a need to manage flux delivery to such instruments in ambiguous conditions where it may be uncertain which instrument will be activated upon a given input command at the surgeon console.

US 2012/202388 A1 discloses a universal connector socket for a surgical apparatus for the connection of different plug connectors of different surgical instruments for RF surgery. The universal connector socket has several plug contact openings for selectively connecting monopolar and bipolar surgical instruments and has several plug contact openings arranged in an overlapping manner for defining an unambiguous plug-in position of a plug connector and a shared reference plug contact opening.

US 2011/045680 A1 discloses a plug system for surgical devices includes a plurality of sockets into which connecting plugs of electrosurgical instruments can be inserted, and a switching matrix having a plurality of switches for connecting at least one of the sockets to at least two different outputs or inputs of an electrosurgery device. A control system having a preselection device for controlling the switching matrix is arranged in the plug system. The switching matrix connects predetermined sockets to predetermined inputs or outputs, depending on data input into the preselection device.

US 2008/020714 A1 discloses a smart connector system includes a machine connector disposed on a face of a surgical machine, an illumination ring located on the face of the surgical machine and disposed around a periphery of the machine connector, an RFID reader antenna located in close proximity to the machine connector and the face of the surgical machine, and an accessory connector adapted to couple with the machine connector. The accessory connector has an RFID tag antenna and is capable of attaching a tool to the surgical machine. When the accessory connector is brought within close proximity to the machine connector, a communications connection is established between the RFID tag antenna and the RFID reader antenna.

US 6 237 604 B1 discloses a medical system for limiting the re-use of a medical probe, such as, e.g., a catheter or a surgical probe, is provided. The medical system includes a medical probe that includes an electronic storage component for storing a probe identification code. The medical probe further includes a disabling circuit, the activation of which disables the electronic storage component. The medical system further includes a control unit, such as, e.g., an RF generator, for connection to the medical probe. The control unit includes control circuit that is configured for automatically reading the probe identification code from the electronic storage component and preventing the medical probe from being operated if the probe identification code is not approved. The control circuit is also configured for determining if the medical probe has been previously used. If the medical probe has been previously used, the control circuit prevents operation of the medical probe and activates the disabling circuitry to disable the electronic storage component in the medical probe. Thus, when the medical probe is reconnected to the control unit, the control circuitry will attempt to read the probe identification code from the disable electronic storage component. Alternatively, the disabling circuitry internally determines whether the medical probe has been previously used and disables the electronic storage component.

US 2009/275940 A1 discloses a surgical tool system with a powered tool that includes a memory in which data describing the characteristics of the power signals that are to be sourced to the toll are stored. A control console based on the data in the tool memory sources the power signal to the tool. Both the data signals written out from the tool memory and the power signals from the console are exchanged over common console terminals and common tool terminals. An isolation circuit in the tool prevents the applied power signals from the console from adversely effecting the tool memory.

### SUMMARY

An aspect of the invention is defined in independent claim 1. Exemplary embodiments are disclosed in the dependent claims. Exemplary embodiments of the present disclosure may solve one or more of the above-mentioned problems and/or may demonstrate one or more of the above-mentioned desirable features. Other features and/or advantages may become apparent from the description that follows.

The present invention provides an electrosurgical energy delivery system, comprising: an electrosurgical instrument comprising: an electronic circuit encoded with first identification data corresponding to the electrosurgical instrument; a data storage device configured to store second identification data corresponding to the electrosurgical instrument, the data storage device being configured to transmit a second data signal including the second identification data; and an instrument connector interface, the instrument connector interface comprising: an instrument data signal transmission connector feature in communication with the electronic circuit to transmit a first data signal including the first identification data, and an instrument electrosurgical energy transmission connector feature that provides an electrosurgical energy pathway for the electrosurgical instrument; and an electrosurgical energy transmission conduit comprising: a conduit data signal transmission pathway, a conduit electrosurgical energy transmission pathway, and a conduit connector interface comprising: a conduit data signal transmission connector feature in communication with the conduit data signal transmission pathway, and a pair of conduit electrosurgical energy transmission connector features in communication with the conduit electrosurgical energy transmission pathway, the pair of conduit electrosurgical transmission connector features being separate from the conduit data signal transmission connector feature, wherein the instrument connector interface and the conduit connector interface are configured to mate with one another to place the electrosurigcal instrument and the electrosurgical energy transmission conduit in data signal transmission communication and electrosurgical energy transmission communication.

Also herein described is an electrosurgical energy delivery system, comprising: an electrosurgical instrument comprising; an electronic circuit encoded with identification data corresponding to the instrument; and a connector interface at the instrument, the connector interface comprising: an instrument data signal transmission connector feature in communication with the electronic circuit to transmit a data signal including the identification data, and an instrument electrosurgical energy transmission connector feature that provides a an electrosurgical energy pathway for the instrument, the electrosurgical energy pathway being a pathway does not form part of the electronic circuit to transmit data; and a surgical flux transmission conduit comprising: a conduit data signal transmission pathway, an conduit electrosurgical energy transmission pathway, and a conduit connector interface comprising: a conduit data signal transmission connector feature in communication with the conduit data signal transmission pathway, and a pair of conduit electrosurgical energy transmission connector features in communication with the conduit electrosurgical energy transmission pathway, the pair of conduit electrosurgical transmission connector features being separate from the conduit data signal transmission connector feature, wherein the instrument connector interface and the conduit connector interface are configured to reversibly mate with one another to place the instrument and transmission conduit in data signal transmission communication and electrosurgical energy transmission communication.

In accordance with an example, the present disclosure contemplates a system for supplying flux that may include a plurality of flux supply pathways, a plurality of remotely-controllable kinematic flux delivery structures operationally coupled to the flux supply pathways to receive flux, a plurality of kinematic support structures, and a control system. The control system may be configured to receive first data identifying which of the remotely-controllable kinematic flux delivery structures is operationally coupled to which of the flux supply pathways and receive second data identifying which of the remotely-controllable kinematic flux delivery structures is operationally coupled to which of the kinematic support structures. In response to an input command signal to deliver flux from a selected one of the remotely-controllable kinematic flux delivery structures, the control system may send a signal to supply flux from one of the flux supply pathways operationally coupled to one of the remotely-controllable kinematic flux delivery structure based on the first data and the second data.

In yet another exemplary embodiment, a system for detecting an operative condition of a surgical instrument of a teleoperated surgical system may include a surgical instrument, a flux transmission conduit, and a sensing device. The surgical instrument may include a housing for coupling the surgical instrument in an installed position at a patient side cart of a teleoperated surgical system. The housing may include a connector feature. The flux transmission conduit may be engageable with the connector feature to place the surgical instrument in a flux connection state. The sensing device may be associated with the patient side cart and disposed to detect a presence of the flux transmission conduit in a flux connection state of the surgical instrument.

Additional objects, features, and/or advantages will be set forth in part in the description which follows, and in part will be obvious from the description, or may be learned by practice of the present disclosure and/or claims. At least some of these objects and advantages may be realized and attained by the elements and combinations particularly pointed out in the appended claims.

It is to be understood that both the foregoing general description and the following detailed description are exemplary and explanatory only and are not restrictive of the claims; rather the claims should be entitled to their full breadth of scope, including equivalents.

### BRIEF DESCRIPTION OF THE DRAWINGS

The present disclosure can be understood from the following detailed description, either alone or together with the accompanying drawings. The drawings are included to provide a further understanding of the present disclosure, and are incorporated in and constitute a part of this specification. The drawings illustrate one or more exemplary embodiments of the present teachings and together with the description serve to explain certain principles and operation. In the drawings,
FIG. 1 is a diagrammatic view of an exemplary teleoperated surgical system in accordance with at least one exemplary embodiment;
FIG. 2 is a schematic block diagram of an exemplary teleoperated surgical system in accordance with at least one exemplary embodiment;
FIG. 3 is a schematic block diagram of an exemplary teleoperated surgical system in accordance with at least one exemplary embodiment;
FIG. 4 is a schematic block diagram of an exemplary teleoperated surgical system in accordance with at least one exemplary embodiment;
FIG. 5 is a flow diagram illustrating an exemplary workflow for controlling flux delivery to a surgical instrument of a teleoperated surgical system in accordance with at least one exemplary embodiment;
FIG. 6 is a perspective view of an exemplary embodiment of a patient side cart;
FIG. 7 is a partial schematic view of an exemplary embodiment of a manipulator arm of a patient side cart with two electrosurgical instruments in an installed position, one of which is shown in electrical communication with a flux generator;
FIG. 8 is a perspective view of an actuation interface assembly at a patient side cart of a teleoperated surgical system in accordance with at least one exemplary embodiment;
FIG. 9A is a perspective view of a connector interface of an energy transmission cable in accordance with one exemplary embodiment;
FIG. 9B is a perspective view of a connector interface of an energy transmission cable in accordance with one exemplary embodiment;
FIG. 9C is a perspective view of another exemplary embodiment of a connector interface of an energy transmission cable;
FIG. 10A is a perspective view of a connector interface of an energy transmission cable in accordance with yet another exemplary embodiment;
FIG. 10B is a perspective view of a connector interface of an energy transmission cable in accordance with another exemplary embodiment; and
FIG. 10C is a perspective view of yet another exemplary embodiment of a connector interface of an energy transmission cable;
FIG. 11 is a front view of a front panel of an electrosurgical generator unit in accordance with at least one exemplary embodiment;
FIG. 12 is a top view of a connector interface of an electrosurgical instrument in accordance with one exemplary embodiment;
FIG. 13 is a top view of a connector interface of another electrosurgical instrument in accordance with one exemplary embodiment;
FIG. 14 is a perspective view of an electrosurgical instrument with a conventional connector interface; and
FIG. 15 is a perspective view of electrosurgical instrument with another conventional connector interface.
FIGS. 16A and 16B are flow diagrams illustrating exemplary workflow components in accordance with various exemplary embodiments of the present disclosure.
FIGS. 17A and 17B are partial cut-away views illustrating an exemplary embodiment of an electrosurgical instrument housing in an installed position at patient side cart, respectively illustrating the electrosurgical instrument not in an energy connection state and in an energy connection state in accordance with the present disclosure.
FIGS. 18A-18C are schematic views depicting three differing states of an exemplary embodiment of a magnet and Hall sensor system utilized for detecting a surgical instrument installation and energy connection state in accordance with the present disclosure.
FIGS. 19A-24 are schematic views of various exemplary embodiments of sensing systems for detecting a surgical instrument installation and/or energy connection state in accordance with the present disclosure.
FIGS. 25A-25C show exemplary embodiments of visual feedback indicators in accordance with the present disclosure

### DETAILED DESCRIPTION

This description and the accompanying drawings that illustrate exemplary embodiments should not be taken as limiting. Various mechanical, compositional, structural, electrical, and operational changes may be made without departing from the scope of this description and the invention as claimed, including equivalents. In some instances, well-known structures, and techniques have not been shown or described in detail so as not to obscure the disclosure. Like numbers in two or more figures represent the same or similar elements. Furthermore, elements and their associated features that are described in detail with reference to one embodiment may, whenever practical, be included in other embodiments in which they are not specifically shown or described. For example, if an element is described in detail with reference to one embodiment and is not described with reference to a second embodiment, the element may nevertheless be claimed as included in the second embodiment.

For the purposes of this specification and appended claims, unless otherwise indicated, all numbers expressing quantities, percentages, or proportions, and other numerical values used in the specification and claims, are to be understood as being modified in all instances by the term "about," to the extent they are not already so modified. Accordingly, unless indicated to the contrary, the numerical parameters set forth in the following specification and attached claims are approximations that may vary depending upon the desired properties sought to be obtained. At the very least, and not as an attempt to limit the application of the doctrine of equivalents to the scope of the claims, each numerical parameter should at least be construed in light of the number of reported significant digits and by applying ordinary rounding techniques.

Surgical instruments that require connection with a flux source may be connected to such a source by a user, for example, a surgeon's assistant or other operating room personnel. As surgery progresses and instruments are installed and removed from the patient side cart, it may become difficult for the personnel to track and determine which instrument will receive flux from a flux source in response to a given input command at the surgeon console. In some teleoperated surgical systems, if an input device is actuated, flux will be supplied from a particular flux source. If a surgical instrument is operationally coupled to that flux source, flux will be transmitted to the instrument, thereby enabling the instrument to deliver the flux to the patient. However, conventional surgical systems are not able to automatically determine which instrument will receive flux upon actuation of a particular input device. Further, if, for example, a plurality of electrosurgical instruments are installed at the patient side cart, conventional surgical systems may prevent two instruments of the same energy type from operating at the same time because some ambiguity may exist as to which instrument will be energized when an energy input command is provided at a surgeon console.

Although for ease of description various exemplary embodiments set forth below describe electrosurgical instruments, electrosurgical energy supply sources, and the delivery of electrosurgical energy (e.g., such as energy for cautery procedures ranging from 100s of volts to 1000s of volts), those having ordinary skill in the art will appreciate that the present disclosure can be applied to a variety of surgical instruments that are provided to deliver various types of flux (e.g., other energy fluxes (such as laser, ultrasound, etc.), a fluid flux, a vacuum pressure flux, smoke evacuation, etc.) by a remotely controlled, external flux generator or other flux supply source to deliver the desired flux to a patient for use in performing, or observing, a surgical procedure. As used herein, the term "flux" may be defined as a flow useful in surgical operations that is transmitted from one source to another source, for example, between a flux supply source and a flux delivery component, such as, for example, an electrosurgical instrument, e.g., to be delivered via end effector thereof.

Nonlimiting examples of types of fluxes encompassed by the present disclosure, with appropriate modification to components using or transmitting the flux may include, for example, electrical energy (e.g., for cautery or nerve stimulation), laser energy, ultrasound energy, or radio frequency energy; fluids (e.g., liquids or gases); image and/or audio streams; vacuum pressure (in which case a negative pressure flux from a vacuum "source" is "delivered" to the instrument), etc. Nonlimiting examples of the flux source may include, for example, energy generators (including, for example, cautery energy and/or nerve stimulation energy generators), fluid delivery sources (e.g., for irrigation), gas supply sources, vacuum sources, etc. By way of nonlimiting example, as will be appreciated by those of ordinary skill in the art, laser energy can be delivered via a fiber optic transmission cable from a laser energy generator to a surgical instrument having an end effector configured to deliver the laser energy to the patient. Further, a flux supply source as used herein can be considered as a sink (e.g., in the case of suction).

Thus, it will be appreciated by one of ordinary skill in the art that the systems and methods described herein with reference to electrosurgical instruments and the delivery of electrical energy are not intended to be limiting and can be used in conjunction with other remotely controlled surgical instruments supplied with remotely delivered fluxes from one or more flux sources. Transmission of the flux from the flux source to the surgical instrument can be via a flux transmission conduit, such as, for example, an electrical energy transmission cable, a hose, a fiber optic cable, etc., configured to be connected to the surgical instrument at one end and to a flux source.

As a safety precaution, some existing surgical systems prohibit the supply of energy (e.g., electrical energy) to instruments installed at the patient side cart if more than one energy delivery surgical instrument is installed. This is because an ambiguity exists regarding which instrument will be activated when an energy input command (e.g., a cautery command) is provided at the surgeon side console. Such prohibition, however, can limit the options available to the surgeon during a surgical procedure and the configuration flexibility of the overall surgical system. For example, it may be desirable to have two electrosurgical instruments installed at the patient side cart, one of which can be used for ordinary grasping and one of which can be used for both grasping and cautery procedures. Installing instruments that have such multiple functionality (e.g., delivering energy and/or grasping as desired) also can permit fewer instrument changes, thereby reducing the overall number of instruments that may need to be stocked. In addition, in certain surgical procedures, it may be desirable to install electrosurgical instruments of differing types, e.g., monopolar and bipolar, based on a requirement of differing cautery operations that may be desired.

Various exemplary embodiments contemplate a teleoperated surgical system in which a surgical instrument, such as an electrosurgical instrument, is mounted at a patient side cart through an actuation interface assembly. The structure of the instrument in combination with the actuation interface assembly, which is attached to a support structure configured to support the instrument at the patient side cart, may be referred to herein as a remotely-controlled kinematic flux delivery structure.

Various exemplary embodiments contemplate a teleoperated surgical system that is able to determine which of a plurality of remotely-controllable kinematic flux delivery structures is operationally coupled to which of a plurality of flux sources in order to determine which of the remotely-controllable kinematic flux delivery structures will be supplied with flux (e.g., energized) when a specific input command is received an input device of a surgeon console. This determination can permit two remotely-controllable kinematic flux delivery structures, including, for example, two electrosurgical instruments of the same energy type to be used at the same time by allowing the system to resolve the ambiguity. Thus, various exemplary embodiments contemplate a way to eliminate potential user errors in correctly tracking which surgical instruments are connected to which flux sources. Various exemplary embodiments contemplate enabling the teleoperated surgical system, based on the above determination and an input command, to supply flux from a particular flux supply source to a specific kinematic flux delivery structure operationally coupled to be actuated in response to the input command.

In accordance with various exemplary embodiments, therefore, because the teleoperated surgical system can determine which of a plurality of surgical instruments installed at an actuation interface assembly are operationally coupled to a specific flux supply source, the system can control the delivery of flux to the various surgical instruments. In particular, because the system is able to associate a specific kinematic structure, which includes a specific surgical instrument at a specific actuation interface assembly to which the instrument is coupled, with a flux supply source, then input devices that provide input commands to supply flux through a flux supply source are able to be unambiguously mapped to the kinematic flux delivery structures.

Various exemplary embodiments also contemplate simplifying the user experience by eliminating the requirement of surgical assistants to manually track which surgical instruments are connected to which flux sources. In addition, various exemplary embodiments contemplate connecting surgical instruments to flux sources using flux transmission conduits that allow the transmission of identification information which identifies the instruments connected with specific flux sources to the teleoperated surgical system.

According to yet another exemplary embodiment, the present disclosure contemplates a method of operationally coupling a surgical flux supply pathway with a surgical flux delivery instrument to supply surgical flux from the surgical flux supply pathway to the surgical flux delivery instrument. The method may include configuring a first connector of a surgical flux transmission conduit to selectively mate with an instrument connector of one of a first surgical flux delivery instrument and a second surgical flux delivery instrument, the instrument connector of the first surgical flux delivery instrument comprising a surgical flux transmission connector feature and a data transmission connector feature, and the instrument connector of the second surgical flux delivery instrument comprising having a surgical flux transmission connector feature and no data transmission connector feature. The method may further include configuring a second connector of the surgical flux transmission conduit to mate with a flux supply source connector of a flux supply source, enabling a supply of surgical flux from the surgical flux supply pathway via the surgical flux transmission conduit, and depending on the selectively mating, selectively enabling transmission of data via the surgical flux transmission conduit.

Yet another exemplary embodiment may include a method of verifying an operational surgical flux communication coupling of a surgical flux delivery instrument to a surgical flux supply pathway that includes transmitting, to a control system, identification data associated with the surgical flux delivery instrument through a data signal transmission pathway created by an operationally-coupled state of the surgical flux delivery instrument and the surgical flux supply pathway.

Various exemplary embodiments provide a robust way for the teleoperated surgical system to determine which one or ones of a plurality of surgical instruments (e.g., electrosurgical instruments) that are installed at a patient side cart are in flux communication (e.g., energy communication) with one or more external flux supply sources (e.g., energy generator (s)). According to various exemplary embodiments, the determination can be made at a controller based on receipt of a signal indicative of a flux transmission conduit being engaged (e.g., an energy transmission cable in electrical connection) with one or more of the installed electrosurgical instruments. Information regarding the determination of which instrument is in flux communication with a flux generator can be shared with the surgeon so that the surgeon knows that if a particular master flux input command at the surgeon side console is provided, which surgical instrument is expected to be activated, e.g., to perform a surgical procedure that uses the delivery of the transmitted flux.

Various exemplary embodiments also contemplate controlling the delivery of a flux, such as, electrical energy, based on the determination information. For example, various exemplary embodiments contemplate prohibiting a flux supply source from sending a flux to one or more electrosurgical instruments upon certain ambiguous conditions of the teleoperated surgical system with respect to which surgical instrument will be activated upon a flux input command at the surgeon side console.

Various exemplary embodiments described herein also provide the ability of the teleoperated surgical system to confirm that an instrument is in an installed position at the patient side cart, e.g., as opposed to being in close proximity or coupled to, but not in an accurately installed position.

In accordance with various exemplary embodiments, therefore, teleoperated surgical systems can in an automated and accurate manner track which installed surgical instruments (e.g., electrosurgical instruments) have flux transmission conduits (e.g., electrical energy transmission cables) connected thereto, confirm the correct installation of the instruments, provide feedback at the surgeon side console to share the information, and control the overall delivery of flux (e.g., electrical energy) to the instruments from a flux supply source (e.g., energy generator) based on the tracking information and master input commands at the surgeon side console. Such automated tracking and control features can, among others, enhance the safety of operation of the teleoperated surgical systems, reduce time spent and potential human error associated with manually tracking the information, reduce time spent in changing instruments at the patient side cart during a procedure that may benefit from the use of two electrosurgical operations by differing instruments, and/or provide greater flexibility in the types of surgical procedures that can be performed and the types of instruments that can be installed at the patient side cart at a time.

### Teleoperated Surgical System

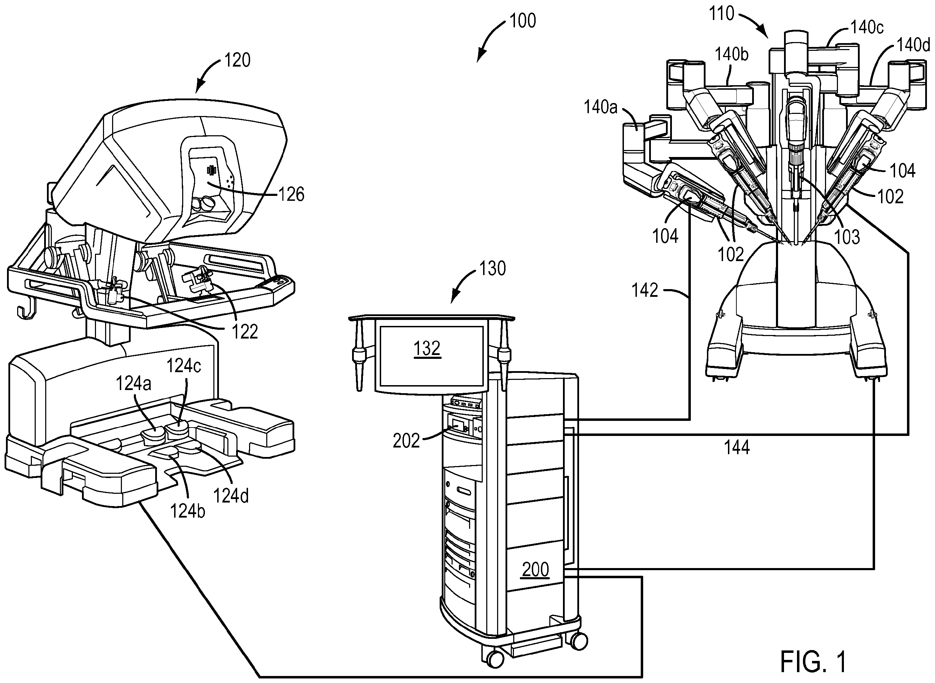

With reference now to FIG. 1, a robotic (teleoperated) surgical system 100 is provided which, in an exemplary embodiment, performs minimally invasive surgical procedures by interfacing with and controlling a variety of remotely operated surgical instruments, such as one or more electrosurgical instruments 102, as those of ordinary skill in the art are generally familiar. The surgical instruments 102 may be selected from a variety of instruments that are configured to perform various surgical procedures, and in accordance with various exemplary embodiments can be electrosurgical instruments, for example, bipolar and/or monopolar electrosurgical instruments. Some surgical instruments can also be so-called mixed mode, which permit the delivery of both monopolar and bipolar energy.

As illustrated in the schematic view of FIGS. 1 the teleoperated surgical system 100 includes a patient side cart 110, a surgeon console 120, and control cart 130. In non-limiting exemplary embodiments of the teleoperated surgical system, the control cart 130 includes "core" processing equipment, such as core processor 200, discussed below, and/or other auxiliary processing equipment, which may be incorporated into or physically supported at the control cart 130. For simplicity, the core processor 200 is referred to herein as a controller at control cart 130. The control cart 130 may also include other controls for operating the teleoperated surgical system. As will be discussed in more detail below, in an exemplary embodiment, signals transmitted from surgeon console 120 may be transmitted to one or more processors at control cart 130, which may interpret the signals and generate commands to be transmitted to the patient side cart 110 to cause manipulation of one or more of electrosurgical instruments and/or patient side manipulators 140a-d to which the electrosurgical instruments are coupled at the patient side cart 110. It is noted that the system components in FIG. 1 are not shown in any particular positioning and can be arranged as desired, with the patient side cart 110 being disposed relative to the patient so as to effect surgery on the patient. A non-limiting, exemplary embodiment of a teleoperated surgical system with which the instruments 102 can be utilized is a da Vinci® Si (model no. IS3000) commercialized by Intuitive Surgical, Inc. of Sunnyvale, California. Reference is also made to U.S. Application Publication No. US 2011/0282358 A1 (published Nov. 17, 2011; entitled "SURGICAL SYSTEM INSTRUMENT MOUNTING"), for a nonlimiting exemplary embodiment of a teleoperated surgical system architecture with which various exemplary embodiments herein can be used.

In general, the surgeon console 120 receives inputs from a user, e.g., a surgeon, by various input devices, including but not limited to, gripping mechanisms 122 and foot pedals 124, etc. and serves as a master controller by which instruments mounted at the patient side cart 110 act as slaves to implement the desired motions of the surgical instrument(s) (e.g., instrument 102), and accordingly perform the desired surgical procedure. For example, while not being limited thereto, the gripping mechanisms 122 may act as "master" devices that may control the electrosurgical instruments 102, which may act as the corresponding "slave" devices at the manipulator arms 140. Further, while not being limited thereto, the foot pedals 124 may be depressed to provide, for example, monopolar or bipolar electrosurgical energy to the instrument 102.

In various exemplary embodiments, suitable output units may include, but are not limited to, a viewer or display 126 that allows the surgeon to view a three-dimensional image of the surgical site, for example, during the surgical procedure, e.g., via an optical endoscope 103 at the patient side cart 110. Other output units may include a speaker (or other component capable of transmitting sound), and/or a component with which a surgeon is in contact that can vibrate or the like to provide haptic feedback. In various exemplary embodiments, the one or more output units may be part of the surgeon console 120 and signals can be transmitted from the control cart 130 thereto. Although in various exemplary embodiments, one or more input mechanisms 122, 124 may be integrated into the surgeon console 120, various other input mechanisms may be added separately and provided so as to be accessible to the surgeon during use of the system, but not necessarily integrated into the surgeon console 120. In the context of the present disclosure, such additional input mechanisms are considered part of the surgeon console.

Thus, a "surgeon console" as used herein includes a console that comprises one or more input devices 122, 124a-d that a surgeon can manipulate to transmit signals, generally through a control cart such as 130 described in more detail below, to actuate a remotely-controllable kinematic structure (e.g., surgical instruments 102 mounted at arms 140) at the patient side cart 110. The surgeon console 120 may also include one or more output devices that can provide feedback to the surgeon. As used herein, it should be understood, however, that a surgeon console can include a unit (e.g., substantially as shown by element 120 in FIG. 1) that integrates the various input and output devices, with, for example, a display, but also can include separate input and/or output devices that are in signal communication with the controllers, such as controllers provided at the control cart and accessible by a surgeon, although not necessarily integrated within a unit with various other input devices. As an example, input units may be provided directly at the control cart 130 and may provide input signals to a processor at the control cart. As such, a "surgeon console" does not necessarily require all of the input and output devices to be integrated into a single unit and can include one or more separate input and/or output devices.

As used herein, the term "controller" and variations thereof should be understood to include one or more controllers (e.g., processors, such as core processor for controlling overall system functionality) that receive, processes and transmit signals to and from the patient side cart 1000 and surgeon side console 2000, as well as to and from one or more energy generators that provide energy to be delivered by an electrosurgical instrument. In accordance with various exemplary embodiments, a control cart as used herein also can include one or more controllers that are provided in direct signal communication with one or more of the surgical instruments 1001 as well. As such, a "control cart" or "controller" does not necessarily require all control processing units to be integrated into a single unit and can include one or more separate control processing units functionally dependent on each other, with various processing functions and capabilities distributed through the entire teleoperated surgical system. Such separate control processing units can be useful to add functionality to operational aspects of a surgical instrument without necessarily having to rely on servo actuators associated with the patient side cart. Such control processing units can also be useful when retrofitting existing teleoperated surgical system as a way to increase control functionality and signal processing into the electronics/control cart.

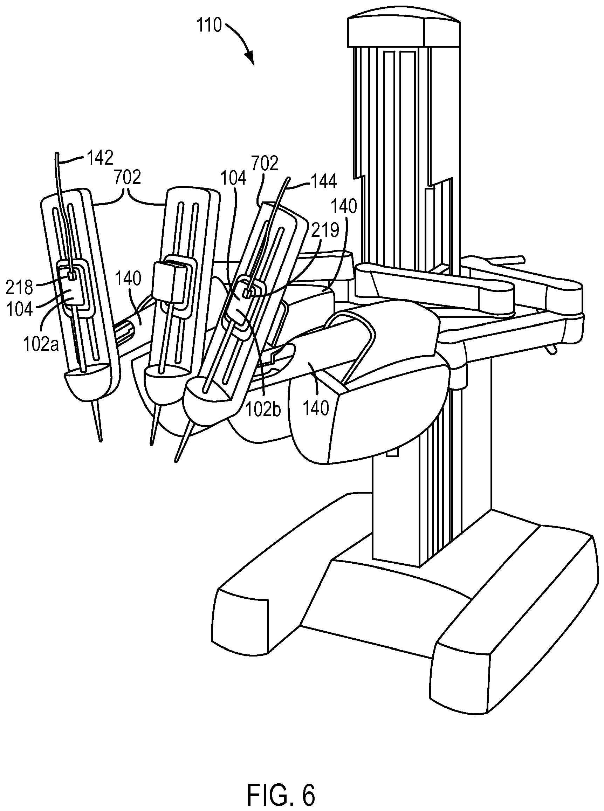

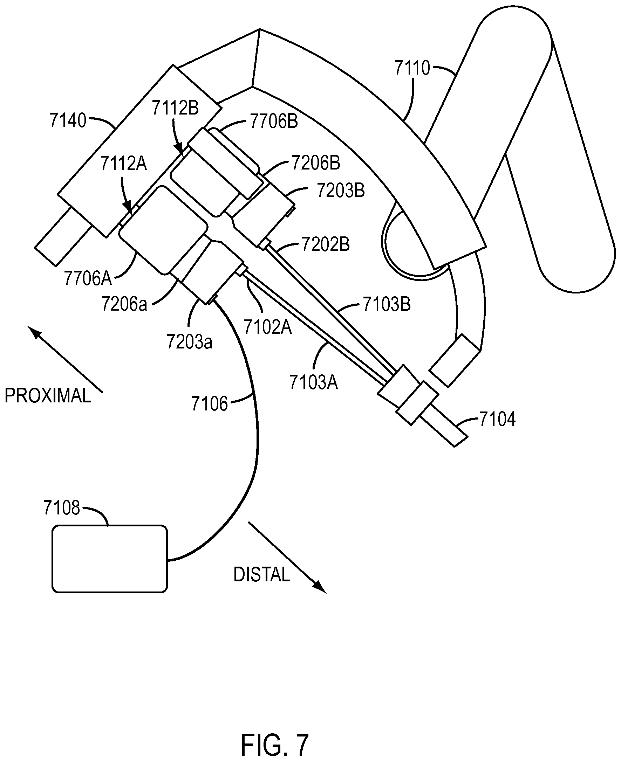

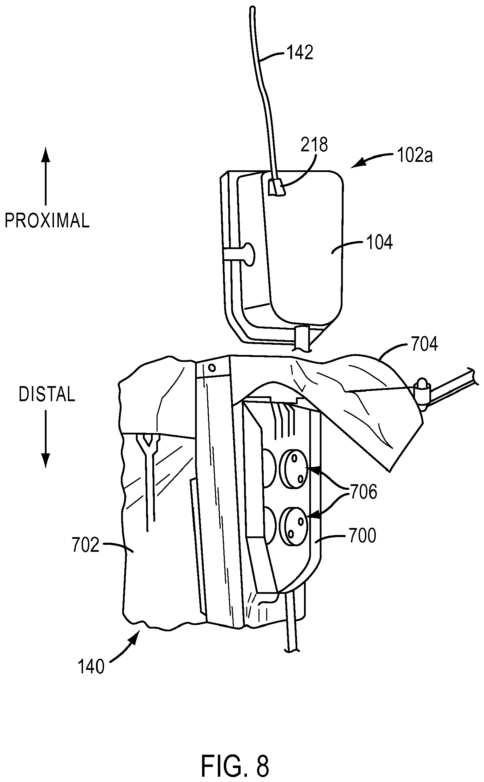

The exemplary embodiments of FIG. 1 and FIG. 6, illustrate a patient side cart 110, with multiple, independently moveable manipulator arms 140 that can support an actuation interface assembly (such as, e.g., 700 shown in FIG. 8) and are configured to hold and manipulate various tools, including, but not limited to, for example, a surgical instrument (e.g., electrosurgical instruments 102), and an endoscope 103. However, those having ordinary skill in the art will appreciate that other patient side cart configurations may be used, such as in the embodiment of FIG. 7. In FIG. 7, a patient side cart may have a single manipulator arm 7110 or single support structure that can support plural surgical instrument actuation interface assemblies 7706a, 7706b mounted on a common base 7140. The actuation interface assemblies interface with transmission mechanisms housed in transmission housings 7203a, 7203b of multiple surgical instruments 7102a, 7102b.

Based on the commands input to input devices at, for example, the surgeon console 120, the patient side cart 110 can position and actuate the instrument(s) 102, 7102 to perform a desired medical procedure via the actuation interface assemblies 706, 7706 at the manipulator arm 140, 7140. The actuation interface assemblies 706, 7706 are configured to engage with transmission mechanisms 104, 7203 provided at a proximal end of the surgical instruments 102 (the "proximal" and "distal" directions being shown in FIGS. 7 and 8 relative to the surgical instrument). The electrosurgical instrument 102 and the actuation interface assembly 706 may be mechanically and electrically connected to be able to operate the instrument 102. According to at least one exemplary embodiment, a drape 704 (shown in FIG. 8) may be provided between the patient side cart 110, particularly over the manipulator arms 140, and the surgical instrument 102 in order to create a sterile boundary between the sterile field, which may include a sterile adapter 700 of the actuation interface assembly 706 to which a sterile surgical instrument 102, 7102 is attached, and the non-sterile patient side cart 110.

A control system receives and transmits various control signals to and from the patient side cart 110 and the surgeon console 120, and can transmit light and process images (e.g., from an endoscope at the patient side cart 110) for display, such as, e.g., display 126 at the surgeon console 120 and/or on a display 132 associated with the control cart 130.

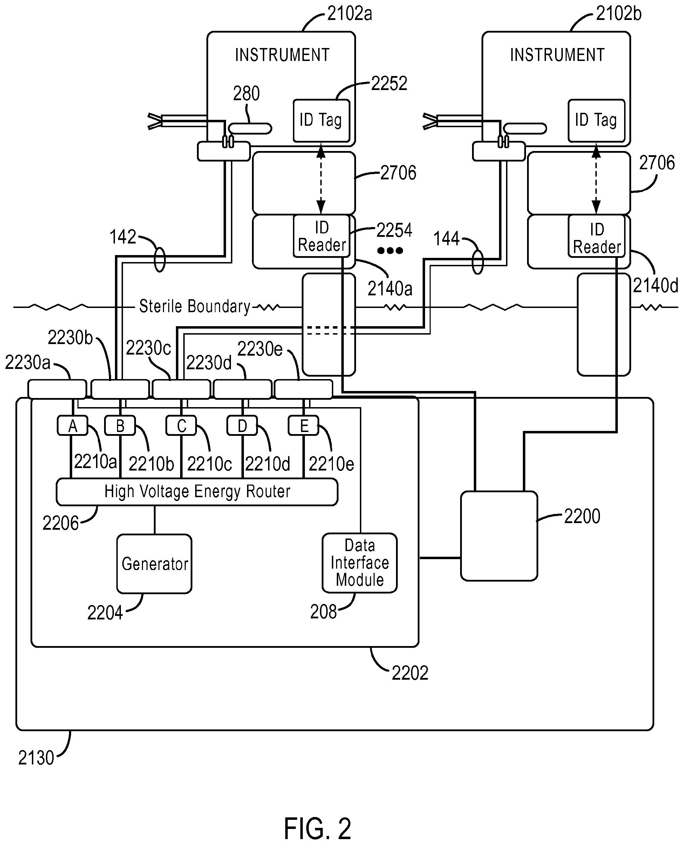

In exemplary embodiments, the control system may have all control functions integrated in one or more processors, such as a core processor 200 at the control cart 130, or, as shown in FIGS. 2-4, additional controllers may be provided as separate units and/or supported (e.g., in shelves) on the control cart 130 for convenience. The latter may be useful, for example, when retrofitting existing control carts to control surgical instruments requiring additional functionality, for example, by providing electrical energy for use in monopolar and bipolar applications. For example, an electrosurgical unit (ESU) 202, which can provide flux sources, such as monopolar and bipolar energy sources, may be provided as separate unit(s) from the core processor 200 and supported on the control cart 130. Alternately, an ESU 202 may be incorporated with the core processor 200 at the control cart 130 into a single integrated flux source and control unit. In various exemplary embodiments, in the case of electrosurgical energy sources for example, one or more electrical energy sources can be used to provide monopolar and/or bipolar energy. Which energy is provided to an electrosurgical instrument (for example, which energy source will be commanded to supply a particular energy type) can be controlled based on mapping particular input devices (e.g., pedals) at the surgeon console to particular energy types (e.g., bipolar or monopolar).

One of ordinary skill in the art would recognize that the controllers, e.g., core processor 200, provided at control cart 130 may be implemented as part of a control system, which, as will be discussed in more detail below, controls various functions of the present disclosure. One of ordinary skill in the art would recognize that functions and features of the controllers, e.g., core processor 200, may be distributed over several devices or software components, including, but not limited to, processors at any of the surgeon console 120, patient side cart 110 and/or other devices, such as ESUs, incorporating processors therein. Functions and features of the control system, which may include core processor 200, may be distributed across several processing devices.

### Flux Disambiguation System and Method

Referring now to FIG. 2, a schematic diagram is depicted which shows a block diagram of exemplary components of an exemplary teleoperated surgical system to deliver flux, e.g., electrical energy, to surgical instruments, such as electrosurgical instruments 2102, in accordance with at least one exemplary embodiment of the present teachings. The control cart 2130 may include at least one processor, e.g., core processor (or controller) 2200 that controls the operation of the electrosurgical instruments 2102 installed at a patient side cart at the patient side manipulators 2140 to which the electrosurgical instruments 2102 are coupled. In an exemplary embodiment, the core processor 2200 can control the delivery of flux (e.g., electrical energy) to the surgical instruments (two such electrosurgical instruments 2102a and 2102b being shown in FIG. 2). While only two electrosurgical instruments 2102a and 2102b are shown and described with reference to FIG. 2, one of ordinary skill in the art would recognize this number is exemplary and the flux disambiguation system would be operable with a single surgical instrument or more than two surgical instruments. Further, the principles of the present teachings would apply irrespective of the number of surgical instruments. In addition, while electrosurgical instruments are shown and described, one of ordinary skill in the art would recognize that other surgical instruments could be mounted at the patient side cart, including surgical instruments capable of supplying other types of flux, including other types of energy, fluid, vacuum pressure, imaging streams, etc. In addition, the principles of the present teachings would apply irrespective of the number of patient side manipulator arms 2140 and the present disclosure may apply to more than two patient side manipulator arms 2140 or a single patient side manipulator arm with multiple actuation interfaces.

The control cart 2130 may include a flux supply source, for example embodied as an electrosurgical unit (ESU) 2202. In various exemplary embodiments, the ESU 2202 may be disposed to transmit to and receive signals from the core processor 2200. In an alternative embodiment, the core processor 2200 and the components of the ESU 2202, which will be discussed further below, can be incorporated together at the control cart 2130 as a single integrated unit, within which at least one of the components of the ESU 2202 may be in communication to receive signals to and from the core processor 2200. As discussed above, the core processor 2200 or other controllers that are part of the control system can be provided at any device and the transmission, reception and processing of signals may be distributed across the core processor 2200 and/or any other processing devices communicating within the teleoperated surgical system, including at the surgeon console 2120, the patient side cart 2110, the control cart 2130, the ESU 2202 or a separate processing unit.

The ESU 2202 includes one or more flux (e.g., electrosurgical energy) generators 2204. For example, one or more electrical energy generators may be provided at the ESU 2202. One or more flux generators 2204 may also be provided separately from one another and/or separately from the ESU 2202. The ESU 2202 may also include, for example, a router, e.g., a high voltage energy router 2206, and a plurality of connector interfaces 2230a-2230e, corresponding to ports 2210a-2210e. A flux source pathway is defined between the one or more flux generators 2204 and the ports 2210a-2210e. The one or more flux generators 2204 are configured to provide flux, for example, electrical energy, such as high voltage cautery energy, to the electrosurgical instruments 2102 through respective ports 2210a-2210e, to which the electrosurgical instruments 2102 are respectively connected. For example, as shown in FIG. 2, electrical energy is provided from the flux generator 2204 to the electrosurgical instrument 2102a through port 2210b and to the electrosurgical instrument 2102b through port 2210c. Although a single flux generator 2204 is depicted in FIG. 2, those having ordinary skill in the art would appreciate that more than one flux source may be included in the system. For example, various flux generators can be used to supply flux, such as electrical energy, to specific types of instruments, such as a bipolar energy generator, a monopolar energy generator, and a harmonic generator. One of ordinary skill in the art would recognize that, for example, a bipolar energy generator would be used to supply energy to an electrosurgical instrument configured to receive bipolar energy, such as bipolar electrosurgical instrument 2102a, and a monopolar energy generator would be used to supply energy to an electrosurgical instrument configured to receive monopolar energy, such as monopolar electrosurgical instrument 2102b. In various exemplary embodiments, a single energy generator may be utilized but controllable, e.g., via the core processor 2200, to provide differing types of energy.

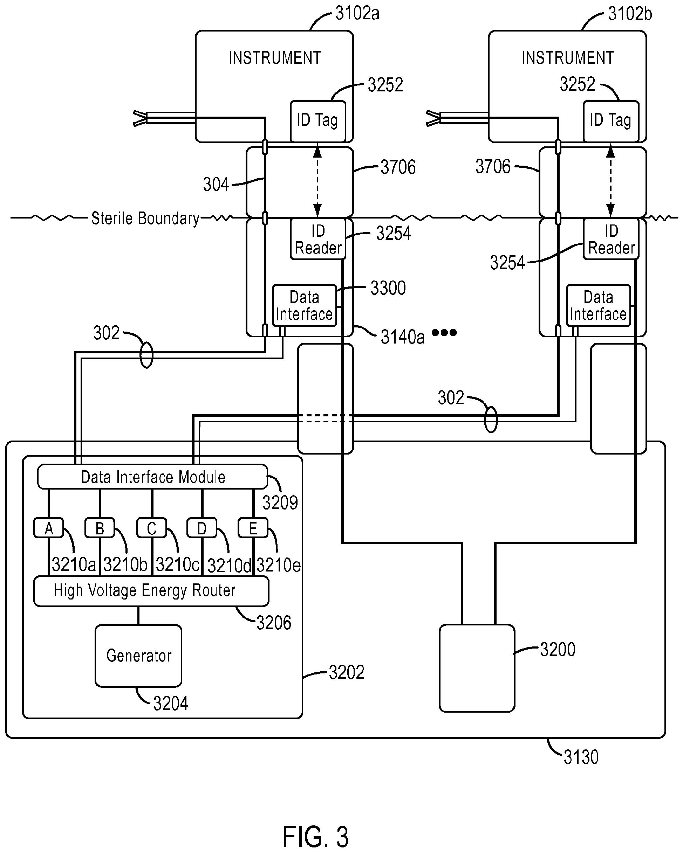

The flux generator 2204 is configured to be placed in operational flux communication with the surgical instruments 2102, which may be provided at the manipulator arms 2140. In various exemplary embodiments, the electrosurgical instrument 2102a is bipolar and configured to be placed in operational communication with the flux generator 2204 through, for example, a flux transmission conduit, such as a bipolar energy transmission cable 142. Also, in an exemplary embodiment, the electrosurgical instrument 2102b is monopolar and configured to be placed in operational communication with the flux generator 2204 through, for example, a flux transmission conduit, such as a monopolar energy transmission cable 144. The ESU 2202 may, in at least one exemplary embodiment of the present disclosure, include one or more data interface modules 208, such as, for example, an instrument identifier interface module 208 (shown in FIG. 2) and/or an actuation interface assembly identifier interface module 208b (shown in FIG. 3), which transmit identification data to the control system and may communicate with, for example, the core processor 2200. As will be described in more detail below, the data interface module 208 is configured to receive identification information of a device, either from a surgical instrument 2102 upon connection of the surgical instrument 2102 with the ESU 2202 or from a support structure upon connection of the ESU with an actuation interface assembly at the support structure to which the surgical instrument 2102 is installed. The data interface modules 208 may be processing devices. The one or more data interface modules 208 may alternately be provided at the control cart 2130 in communication with the core processor 2200 and in the same unit with the core processor 2200.

The flux generator 2204 may be connected to the router 2206. At least one of the surgical instruments 2102 is in communication via a flux transmission conduit, such as energy transmission cables 142, 144, with the flux generator 2204 through one of the ports 2210a-2210e. In various exemplary embodiments, as will be described in more detail below, when the surgeon (e.g., at the surgeon console 2120) provides a flux input command via input devices, a controller of the control system sends a signal to supply flux through a specific one of the ports 2210a-2210e via the flux transmission conduit 142, 144 to the surgical instrument 2102 connected to the energized port. The router 2206 may, in an exemplary embodiment, route flux, e.g., electrical energy such as high voltage cautery energy, from one or more flux generators 2204 to the respective instruments 2102a, 2102b through one of the ports 2210a-2210e.

According to at least one exemplary embodiment of the present disclosure, a plurality of connector interfaces 2230a-2230e is provided at, for example, the electrosurgical unit 2202. The connector interfaces 2230a-2230e are configured to interface with the flux transmission conduits, e.g. the bipolar energy transmission cable 142 or the monopolar energy transmission cable 144, operationally coupled to one of the surgical instruments 2102, e.g., electrosurgical instruments, at the patient side cart 2110. In an exemplary embodiment, specific ports, e.g., ports 2210a-221 0b, may include a connector interface (described in more detail below) configured to connect with specific surgical instruments, such as the bipolar electrosurgical instrument 2102a, by the bipolar energy transmission cable 142. Electrical energy may be provided from the flux generator 2204, e.g., a bipolar energy generator, through one of the ports 2210a-2210b through the bipolar energy transmission cable 142 to the bipolar electrosurgical instrument 2102a. Ports 2210c-2210d, for example, may include a connector interface configured to connect with specific surgical instruments, such as the monopolar electrosurgical instrument 2102b, by the monopolar energy transmission cable 144. Electrical energy may be provided from the flux generator 2204, e.g., a monopolar energy generator, through one of the ports 2210c-2210d through the monopolar energy transmission cable 144 to the monopolar electrosurgical instrument 2102b. Those having ordinary skill in the art will appreciate that the ports could have numerous configurations and be arranged and/or distributed among one or more flux supply sources.

In accordance with at least one exemplary embodiment of the present disclosure, each of the specific surgical instruments 2102, e.g., electrosurgical instruments 2102a, 2102b, includes a unique identifier identifying the specific electrosurgical instrument 2102a, 2102b. The unique identifier may be, for example, a unique serial number for the specific electrosurgical instrument 2102a, 2102b. The unique identifier may be encoded at the electrosurgical instrument 2102 at a readable or readable and writable memory structure. According to one exemplary embodiment of the present disclosure, a unique identifier is encoded at an electronic circuit, such as an EPROM or EEPROM electronic chip 280, disposed at the surgical instrument 2102. In addition, according to at least one exemplary embodiment of the present disclosure, the surgical instrument 2102 includes a transmitter, e.g., a radio frequency identification (RFID) tag 2252, at which is also encoded the unique identifier. According to exemplary embodiments of the present disclosure, each of the manipulator arms 2140a-2140d includes a receiver, such as an RFID reader 2254, configured to sense the information transmitted from the RFID tag 2252 corresponding to an electrosurgical instrument 2102. The transmitter and the receiver may both support various wireless communication protocols, with which those of ordinary skill in the art would be familiar.

One of ordinary skill in the art would recognize that the receiver, such as the RFID reader 2254, may be provided at any structure to which the actuation interface assembly is attached. For example, the actuation interface assembly 706 of FIG. 8 may be connected to a manipulator arm through a support structure 702 (also shown in FIG. 8) at the arm, or may be provided at a drape 704 (shown in FIG. 8) attached to one of the manipulator arms or at an adapter 700 at one of the manipulator arms.

The surgical instrument 2102 is installed at an actuation interface assembly 2700, which may be attached to one of the manipulator arms 2140. One of the flux transmission conduits, e.g., energy transmission cables 142, 144, can connect the surgical instrument 2102 that is installed at the actuation interface assembly 2706 (or at a sterile adapter (e.g., sterile adapter 706) associated with an actuation interface assembly) to a flux supply pathway, e.g., through ports 2210a-2210e connected with the one or more flux generators 2204 at the electrosurgical unit 2202 or at ports at the control cart 2130 individually connected to separate flux generators, through a sterile boundary.

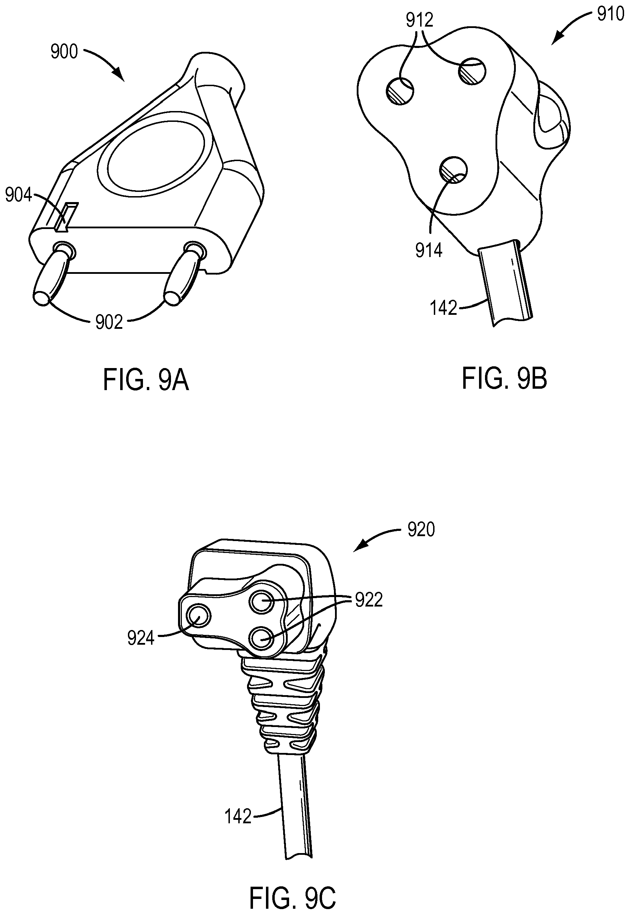

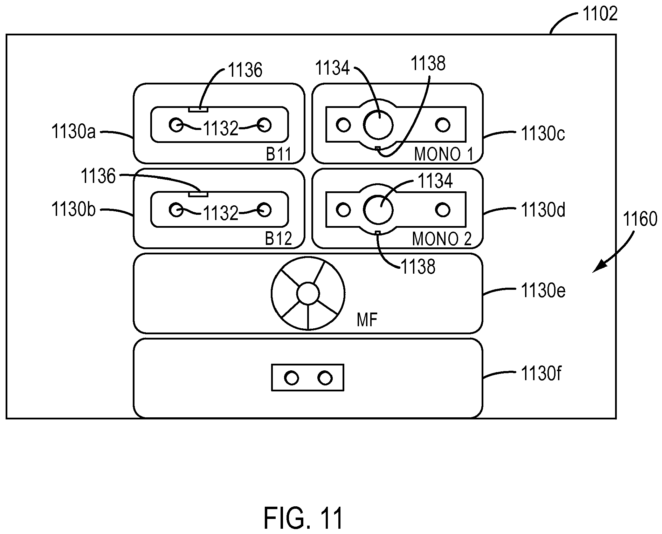

When the energy transmission cables 142, 144 connect the surgical instruments 2102 through the flux supply pathway at the electrosurgical unit 2202 or the control cart 2130, an instrument identification signal indicative of the unique identifier that is encoded at, for example, an electronic chip 280 of the instrument, is output to one of the flux source connector interfaces 2230a-2230e corresponding to the ports 2210a-2210e. The first instrument identification signal may be transmitted through a data transmission line of the various energy transmission cables 142, 144 in accordance with the present disclosure and described in more detail below. For example, as set forth further below, a data transmission terminal 221 or 225 (see FIGS. 9A and 11A) of the energy transmission cables 142, 144 may output the first instrument identification signal. The first instrument identification signal may be read by the instrument identifier interface module 208, which determines the unique identifier of the specific electrosurgical instrument 2102a from the first instrument identification signal. In an alternative embodiment, the first instrument identification signal may be provided directly to the core processor 2200 along with, for example, information regarding the flux supply pathway, e.g., through ports 2210a-2210e, to which the surgical instrument 2102 is operationally-coupled. The first instrument identification signal may also include other information, such as a flux delivery type of the surgical instrument 2102. The flux delivery type may indicate, for example, the type of flux the surgical instrument 2102 transmits, e.g., electrical energy, fluid, vacuum pressure, etc. and may also identify, for example, whether the instrument 2102 requires bipolar or monopolar energy.

Upon receiving and reading the first instrument identification signal through a specific connector interface 2230a-2230e corresponding to a specific port 2210a-2210e, the instrument identifier interface module 208 may identify which one of the flux supply pathways, e.g., through ports 2210a-2210e, is operationally-coupled with the specific instrument 2102 that transmitted the first instrument identification signal. For example, as shown in FIG. 2, the instrument identifier interface module 208 may identify that port 2210b is operationally-coupled to the bipolar electrosurgical instrument 2102a. One of ordinary skill in the art would recognize that the instrument identifier interface module 208 need not be disposed within ESU 2202 and may be provided separately from ESU 2202, for example, incorporated into another processing unit, such as core processor 2200, etc. The instrument identifier interface module 208 may provide the associated data, which identifies the operationally-coupled pair of the surgical instrument and the port 2210a-2210e, to the core processor 2200.

When the electrosurgical instrument 2102a is installed at an actuation interface assembly 2706 at a specific manipulator arm 2140, a second instrument identification signal is output from, for example, the RFID tag 2252 associated with the electrosurgical instrument 2102a and is read by the RFID reader 2254. The RFID reader 2254 provides instrument identification information to, for example, the core processor 2200 identifying a specific surgical instrument 2102. When the RFID reader 2254 provides the instrument identification information to the core processor 2200, the core processor 2200 recognizes that the instrument identification information is associated with a specific actuation interface assembly 2706. Any structure to which the actuation interface assembly 2706 is coupled, e.g., other kinematic support structures, such as the manipulator arm 2140 or alternatively the drape 704 or the adapter 700 in the embodiment of FIG. 8, etc., may be provided with the reader 2254 that reads the instrument identification signal. The control system is therefore able to determine that the instrument identification signal is associated with a specific actuation assembly that is coupled to the structure that read the instrument identification signal. For example, as shown in FIG. 2, when the electrosurgical instrument 2102a is installed at the manipulator arm 2140a, the RFID reader 2254 at the manipulator arm 2140a reads the output instrument identification signal from the electrosurgical instrument 2102a and notifies the core processor 2200 that the electrosurgical instrument 2102a is installed at an actuation interface assembly 2706 at the manipulator arm 2140a.

The control system therefore receives information that identifies that a specific surgical instrument 2102 is operationally-coupled to a specific flux supply pathway, e.g., from one of the flux generators 2204 through one of ports 2210a-2210e. The control system also receives information that the specific surgical instrument 2102 is associated with a specific actuation interface assembly. Thus, the control system is able to identify that a remotely-controllable kinematic flux delivery structure, which includes the identified surgical instrument 2102 coupled to a specific actuation interface assembly at, for example, manipulator arm 2140, is operationally-coupled to a particular flux supply pathway, e.g., through one of the ports 2210a-2210e.

In addition, the control system recognizes which of the input devices at the surgeon console is operationally coupled to a specific kinematic structure. For example, the control system maps one of the input devices to a structure to which the actuation interface assembly is coupled. Therefore, because the specific surgical instrument 2102 has been identified as being coupled to the actuation interface assembly 2706 coupled with a specific structure, e.g., manipulator arm 2140, the control system is able to determine that a particular remotely-controllable kinematic flux delivery structure is operationally coupled to the input device that has been mapped to the specific structure to which the actuation interface assembly 2706 is coupled. Thus, when flux is selected to be supplied to the identified surgical instrument 2102 of a specific remotely-controllable kinematic flux delivery structure, then the control system sends a signal to cause flux to be supplied through a particular port, e.g., ports 2210a-2210e, that is recognized as being operationally-coupled to the specific remotely-controllable kinematic flux delivery structure.

The core processor 2200 may provide a routing signal to the router, e.g., energy router 2206, to direct the energy router 2206 to route energy through the respective port (e.g., port 2210b, 2210c) determined to be operationally-coupled with the respective instruments 2102a, 2102b installed at the actuation interface assemblies 2706 of the respective arms 2140a-2140d. Thus, as the control system is able to indicate which port 2210a-2210e should be provided with energy based on which actuation interface assemblies 2706 at one of arms 2140a-2140d a specific electrosurgical instrument 2102 is installed, then the input devices 124a-124d can be mapped to a specific electrosurgical instrument 2102 installed at an actuation interface assembly 706 at a particular manipulator arm 2140a-2140d.

In various exemplary embodiments, some of the input devices 124 can be assigned by the core processor 2200 to operate functions (e.g., monopolar, bipolar) of the instruments, e.g, electrosurgical instrument 2102a, 2102b that are currently installed and being controlled by a user at the surgeon console 120. Such mapping of the input device at the surgeon console 120 to perform functions of instruments at the patient side car can be either functional or positional. In the former, for example, a particular foot pedal 124a-124d is assigned to cause bipolar energy to be supplied from the electrical flux supply source (e.g., ESU). In the latter, for example, a left bank of foot pedals 124a, 124b is assigned to cause energy delivery to an instrument controlled by the left gripping input device 122 of the surgeon side console and the right bank of pedals 124c, 124d to operate the energy function of an instrument controlled by the right gripping input device 122. For this and other positional mapping that can be utilized for mapping of input devices at the surgeon side console to instruments at the patient side cart, reference is made to U.S. Patent Application No. 14/028,006, publication no. US 2014-0081455 A1, filed September 16, 2013 (for "METHODS AND SYSTEMS FOR ASSIGNING INPUT DEVICES TO TELEOPERATED SUGICAL INSTRUMENT FUNCTIONS"), which claims priority to non-published U.S. Provisional Application No. 61/702,166, filed September 17, 2012. Thus, for example, because the system recognizes that the electrosurgical instrument 2102a is installed at the actuation interface assembly 2706 coupled to a particular manipulator arm 2140a and recognizes the instrument type, then based on the mapping, the system is able to provide the correct type of energy to the instrument 2102a upon receiving a command from whichever pedal 124a-124d is determined to be mapped to the instrument 2102a.

When the core processor 2200 associates one of the ports 2210a-2210e with an actuation interface assembly to which a specific electrosurgical instrument 2102 is coupled, the control system can provide feedback to a user indicating the operationally coupled pair of the remotely-controllable kinematic flux delivery structure and the specific port 2210a-2210e, causing the feedback to be output, for example, at the display 126 and/or display 132. The output can include, for example, the location where the instrument is installed and the type of the instrument (e.g., bipolar or monopolar).

Turning now to FIG. 3, in accordance with at least one exemplary embodiment of the present disclosure, instead of energy transmission cables 142, 144 directly connecting the electrosurgical unit 2202 to the surgical instrument 2102, as in the exemplary embodiment of FIG. 2, an energy transmission cable 302 may extend from the electrosurgical unit 3202 through the manipulator arms 3140a to instrument 3102a. Similar to the energy transmission cables 142, 144, the energy transmission cable 302 includes an electrical energy transmission line to provide energy between a flux generator 3204 and the surgical instrument 3102. The energy transmission cable 302 also includes a data signal transmission line to provide data identifying, for example, the manipulator arm 3140 to which an identified surgical instrument 3102 is operationally coupled by an actuation interface assembly 3706. The identification data is provided to a data interface module 3209, which may be provided at the electrosurgical unit 3202 or at the control cart 3130, and which may communicate with a processing device of the control system, such as core processor 3200. One or more auxiliary cables 304 may be disposed in each of the manipulator arms 3140 through to the actuation interface adapter 3700 or, in an alternative embodiment, may be disposed through a sterile drape to the actuation interface adapter 3700. The auxiliary cables 304 are configured to be placed in flux communication with the energy transmission cable 302.

Similarly to the embodiment disclosed in FIG. 2, a unique identifier is encoded at each of the instruments 3102 and a transmitter 3252 transmits an instrument identification signal which is read by the receiver 3254. The receiver 3254 in an exemplary embodiment may be disposed at the manipulator arm 3140a, 3140b or at actuation interface assemblies 3700. The receiver 3254 may output an instrument identification signal, which can be received by the core processor 3200. In an installed position at an actuation interface assembly 3700, an electrosurgical instrument 3102 is thus operationally coupled to a flux supply source, for example, the electrosurgical unit 3202, through the cables 302 and 304. In accordance with exemplary embodiments of the present disclosure, the energy transmission cable 302 is connected between the flux generator 3204, for example, of the electrosurgical unit 3202 via one of the ports 3210a-3210e. The cable 302 also is connected to a manipulator arm 3140, and ultimately at the actuation interface assembly 3700, to which the surgical instrument 3102 is operationally coupled. A data interface 3300 at the manipulator arm 3140 identifies the manipulator arm 3140 to which the flux transmission conduit, e.g., energy transmission cable 302, is connected and provides manipulator arm identification information to a data interface module, such as manipulator arm interface module 3209, at the electrosurgical unit 3202 or at the control cart 130 and in communication with, for example, the core processor 3200. The data interface module 3209 provides information to the core processor 3200 identifying the respective manipulator arm 3140a and the port 3210b, to which the manipulator arm 3140a is connected by the energy transmission cable 302. The core processor 3200 then is able to associate the identified instrument 3102, e.g., instrument 3102a installed at an actuation interface assembly 3700 at, for example, the identified manipulator arm 3140a, with the port 3210b operationally coupled to the identified manipulator arm 3140a. Thus, the processor 3200 can determine the port 3210b through which flux should be supplied upon receipt of an input command to energize the instrument 3102a. Thereafter, in response to an input command signal at one of the input devices of a surgeon console (e.g., input device 124) intended for a specific kinematic flux delivery structure, including a specific surgical instrument 3102a operationally coupled with an actuation interface assembly coupled to an identified manipulator arm 3140a, the control system can send a signal to supply flux from the port 3210b that is operationally coupled to the specific kinematic structure.

In accordance with another exemplary embodiment of the present disclosure, instead of providing the identification of the manipulator arm 3140 to the data interface module 3209 at, for example, the electrosurgical unit 3202, one of ports 3210a-3210e, could be identified to the data interface 3300 at the manipulator arm 3140. For example, port 3210b could provide a port identification through the data signal transmission line of the energy transmission cable 302 to the manipulator arm 3140a or any other structure to which the actuation interface assembly is coupled, and the manipulator arm 3140a could provide data to, for example, the core processor 3200 indicating the instrument identification of, e.g., electrosurgical instrument 3102a and the port identification of, e.g., port 3210b. In accordance with an exemplary embodiment, the core processor 3200 then can associate the identified instrument 3102a installed at the actuation interface assembly at the specific manipulator arm 3140a (i.e., the remotely-controllable kinematic flux delivery structure) that provided the instrument identification with the port 3210b in communication with the manipulator arm 3140a including the actuation interface assembly to determine the port 3210a-3210e, e.g., port 3210b, to which the instrument 3102, e.g., instrument 3102a, is electrically connected. Thereafter, in response to an input at one of the input devices 124, the core processor 3200 can output a signal to supply flux from the port 3210a-3210e, e.g., port 3210b, that is operationally coupled to a selected kinematic structure (including the surgical instrument 3102 operationally coupled to the actuation interface assembly). The signal may cause flux to be supplied by routing the flux from a flux generator 3204 through one of the ports 3210a-3210e.

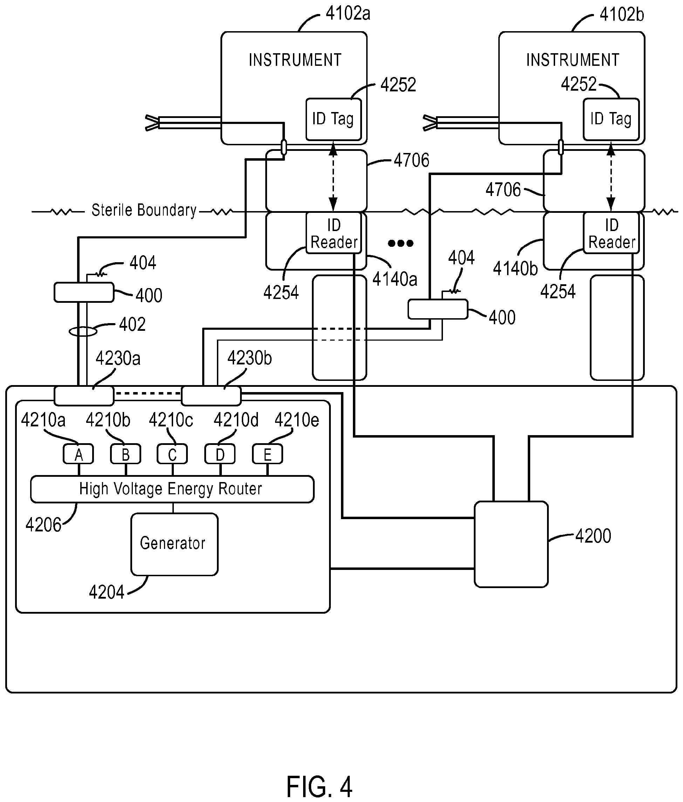

Referring now to FIG. 4, according to another exemplary embodiment, an alternative connection scheme between a flux transmission conduit and a surgical instrument. FIG. 4 illustrates a flux transmission conduit, such as energy transmission cable 402, for example, that is disposed through a sterile drape (such as drape 704 in FIG. 8) and is connected at a drape connection point 400 to a connector, e.g., a socket (not shown). An energy transmission cable 402 also extends to the actuation interface assembly 4706 to which the surgical instrument 4102 is attached. With reference to FIG. 8, the drape connection points 400 can include a socket disposed on a support structure 702 to which the actuation interface assemblies 700 are mounted. Each socket at the respective support structures can have a different value resistor that can be detected. For example, with reference again to FIG. 4, a presence detection resistor 404 at the drape connection point 400 can detect the resistance value and provide an identification through the data signal transmission line of the energy transmission cable 402 of the support structure (e.g., support structure 702) provided at manipulator arm, e.g., arm 4140a, to which the drape (e.g., drape 704 in FIG. 8) is connected at the drape connection point 400. The identification information of the support structure 702 may be provided directly to the core processor 4200 with information as to the port 4210a to which the drape 704 housing the energy transmission cable 402, which is connected with the manipulator arm 4140, is connected. Alternately, the identification information may be provided to a data interface module (not shown in FIG. 4; see FIGS. 2 and 3), which may be provided at, for example, the electrosurgical unit 4202. In addition, similarly to the embodiments disclosed in FIGS. 2 and 3, a unique identifier is encoded at each of the instruments 4102 and a transmitter 4252 transmits an instrument identification signal which is read by a receiver 4254, for example, coupled to an arm 4104 or support structure on an arm 4104. An instrument identification signal is output from the receiver 4254 to the control system, including, e.g., the core processor 4200. The control system then associates the identified instrument, e.g., instrument 4102a, with the identified arm, e.g., arm 4140a, and the port, e.g., port 4210b, connected to the arm 4140a by the energy transmission cable 402 within the drape. Thereafter, in response to an input at one of the input devices, such as input devices 124, the control system can output a signal to cause flux to be supplied through a particular port, e.g., port 4210b, and one of a corresponding interface, e.g., one of interface 4230a...4230b,to a selected kinematic flux delivery structure (which includes the identified instrument coupled to an actuation interface assembly coupled to, for example, the identified arm 4140a).

One of ordinary skill in the art would recognize that the information indicative of the association between the above-described elements (e.g., the electrosurgical instrument, the flux supply pathway (including the ports), the arm or other support structure to which the actuation interface assembly is coupled, etc.) may be transmitted to the core processor 200, 2200, 3200, and 4200 at control cart 130, 2130, 3130, and 4130 or may be distributed across multiple control devices, including, but not limited to, processors at the surgeon console 120, processors at the patient side cart 110, processors provided at or in communication with control cart 130, 2130, 3130, and 4130 or core processor 200, 2200, 3200, and 4200 at control cart 130, 2130, 3130, and 4130, etc. One of ordinary skill in the art would recognize that the information indicative of the association between the above-described elements may occur at any of the described processing or control elements, collectively referred to as a control system.

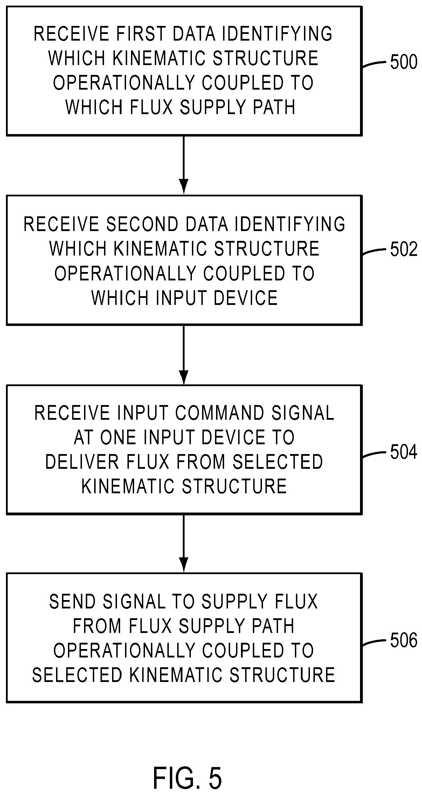

Turning now to FIG. 5, the teleoperated surgical system 100 as shown and described in FIG. 1 and in the exemplary embodiment of FIG. 2 is used to illustrate an the implementation of the present disclosure, which contemplates providing a method and system for delivering flux, e.g., electrical energy, fluid, etc. to a surgical instrument 102, e.g., an electrosurgical instrument. FIG. 5 is an exemplary embodiment of a workflow for determining which of a plurality of flux supply pathways is to be supplied with flux, which is dependent on a selected kinematic structure to which the flux supply pathway is operationally coupled. In various exemplary embodiments in accordance with the present disclosure, in the exemplary workflow of FIG. 5, at operation 500 first data is received by, for example, the control system, that identifies which of a plurality of remotely-controllable kinematic flux delivery structures, which includes the surgical instrument 102 operationally coupled to an actuation interface assembly at a manipulator arm 140 of a patient side cart 110, is operationally coupled to which of a plurality of flux supply pathways, e.g., through ports 2210a-2210e. At operation 502, second data may be received at the control system that identifies which of the remotely-controllable kinematic flux delivery structures is operationally coupled to which of the plurality of input devices at the surgeon console. The input devices may be mapped in advance to operate functions that are available from the instrument(s) 102 being controlled (such mapping can include, for example, functional or positional mapping), and the control system is able to identify which remotely-controllable kinematic flux delivery structure is operationally coupled to which of the input devices. For exemplary positional mapping techniques that can be utilized, reference is made to U.S. Patent Application No. 14/028,006, publication no. US 2014-0081455 A1, filed September 16, 2013 (for "METHODS AND SYSTEMS FOR ASSIGNING INPUT DEVICES TO TELEOPERATED SUGICAL INSTRUMENT FUNCTIONS"), and non-published U.S. Provisional Patent Application No. 61/702,166, filed September 17, 2012.

At operation 504, an input command signal is received at one input device to deliver flux from a selected remotely-controllable kinematic flux delivery structure. Then, at operation 506, a signal is transmitted to supply flux from the respective flux supply pathway, e.g., through one of the ports 2210a-2210e, that is operationally coupled to the selected remotely-controllable kinematic flux delivery structure. The control system transmits the signal to supply flux after determining which of the flux source pathways, e.g., one of the flux generators 2204 in communication with one of the ports 2210a-2210e, is operationally coupled to which of the kinematic flux delivery structures in order to cause flux to be supplied from a flux generator through the appropriate port 2210a-2210e to a selected remotely-controllable kinematic flux delivery structure that is operationally coupled to one of the plurality of input devices that generate input command signals to control the remotely-controllable kinematic flux delivery structures.

One of ordinary skill in the art would recognize that when data is received by a control system, the data may be received at one or more of the controllers or processors described above as part of the control system. Further, the receipt of and/or processing of the data may be distributed across one or more of the controllers or processors of the control system.

### Flux Transmission Conduits and ESU Interface

In accordance with various exemplary embodiments, flux transmission conduits are provided that connect a flux delivery device, e.g., electrosurgical instrument 102, and a flux supply source. The flux transmission conduits are provided with a flux delivery transmission line, which provides for the transmission of flux from the flux supply source to the flux delivery device. In addition, the flux transmission conduits are also provided with a data signal transmission line that provides for the transmission of data, such as flux delivery device identification data, from the flux delivery device through a flux supply pathway connector interface to a data signal processor, e.g., data interface module 208. Thus, in providing the identification data, the flux transmission conduit facilitates the ability of the control system to determine, through the reception of the identification data, an association between the kinematic flux delivery structure (including the flux delivery device) and a flux supply pathway (e.g., from the flux supply source through a port). The association between the kinematic flux delivery structure and the flux supply pathway enables the system to determine from which of the flux supply pathways that flux is to be supplied when flux is intended for a specific kinematic flux delivery structure.

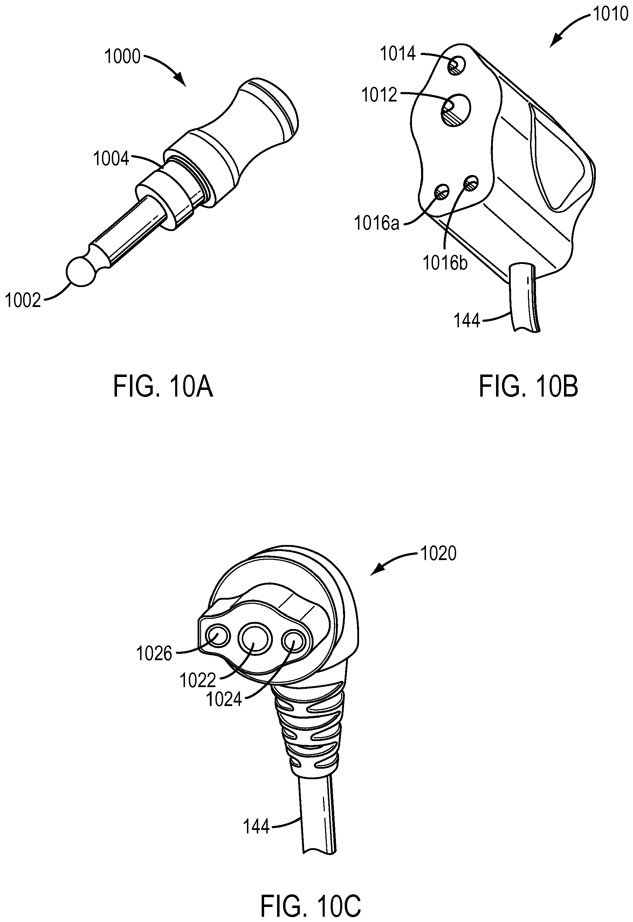

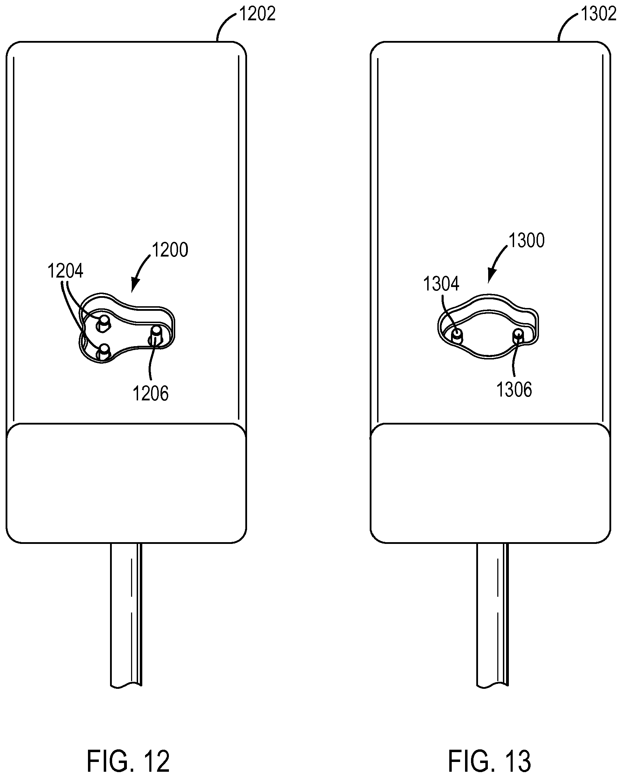

FIGS. 9A-9C show exemplary embodiments of flux supply source connector interfaces of a flux transmission conduit (conduits 142 partially shown in FIGS. 9B and 9C) in accordance with exemplary embodiments, and FIGS. 10A-C show exemplary embodiments of surgical connector interfaces of a flux transmission conduit (conduits partially shown in FIGS. 10B and 10C) in accordance with other exemplary embodiments. FIG. 11 shows an exemplary embodiment of a front panel of flux supply source connector interfaces. FIGS. 12 and 13 show exemplary embodiments of flux delivery instrument connector interfaces. While various exemplary embodiments herein describe flux transmission conduits that transmit electrosurgical energy, e.g., cautery energy, one of ordinary skill in the art would recognize that flux transmission conduits in accordance with the present disclosure can transmit any of a variety of fluxes, including, but not limited to laser energy, ultrasound energy, fluids, gases, radio frequency energy, nerve stimulation energy (e.g., for nerve identification and/or causing associated muscle contraction) image and/or audio streams, vacuum pressure, etc., with modifications made to the conduit structure and/or connectors as those having ordinary skill in the art would appreciate.

According to an exemplary embodiment, flux transmission conduits can include both a flux transmission line and a data signal transmission line, with the latter being configured to transmit data, for example from a memory chip. Also, in various exemplary embodiments, as discussed above with reference to FIGS. 2-5, the flux transmission line may be an electrical energy transmission cable configured to delivery bipolar or monopolar electrical energy, for example.

In an exemplary embodiment, the flux transmission conduit may be a bipolar energy transmission cable, such as bipolar energy transmission cable 142. FIG. 9A shows a first connector interface 900 configured to connect with flux supply source connector interfaces 1130a, 1130b (shown in FIG. 11), which in an exemplary embodiment may be provided at an electrosurgical unit (ESU) 1102. FIGS. 9B and 9C show different configurations of another connector interface 910, 920 of the bipolar energy transmission cable 142 configured to connect with a bipolar electrosurgical instrument 1202 at an instrument connector interface 1200 (shown in FIG. 12).

As shown in FIG. 9A, the connector interface 900 includes two male flux transmission terminals 902, configured to be placed in electrical energy contact with female terminals of a connector interface of a flux supply source, such as flux supply source 1102 of FIG. 11. The connector interface 900 is configured to engage with a corresponding flux source connector interface to transmit flux from the flux supply source through the cable 142. In addition to the terminals 902, the connector interface 900 includes a data signal transmission terminal 904, which may be, for example, an electrical pad, such as a copper pad. While a single data signal transmission terminal 904 is shown in FIG. 9A, two data signal transmission terminals, for example, two copper pads, may be provided on the top and the bottom of the connector interface 900 to allow for connection to a corresponding connector interface at, for example, an electrosurgical unit in either orientation in which the connector interface 900 would be in a connected state. The data signal transmission terminal 904 transmits identification data from, e.g., from a bipolar electrosurgical instrument 1202 (shown in FIG. 12) t o a data signal processor, e.g., such as data interface module 208 or 309. Specifically, the data signal transmission terminal 904 is an interface of a data signal transmission line extending through the bipolar energy transmission cable 142. When the bipolar energy transmission cable 142 interfaces with, for example, an electrosurgical instrument that is provided with identification data, the identification data is provided from the electrosurgical instrument through the data signal transmission line to the data signal transmission terminal 904.

Referring to FIG. 11, in an exemplary embodiment, a front panel 1160 of a flux supply source 1102, e.g., ESU, is provided with a plurality of flux supply source connector interfaces 1130a-1130e configured to interface with a connector interface of an energy transmission cable. Each of the flux source connector interfaces 1130a-1130e corresponds with a specific port, e.g., one of ports 2210a-2210e, 3210a-3210e, or 4210a-4210e. Connector interfaces 1130e and 1130f may be, for example, a standard electrical connection interface 1130e or a patient return connection interface 1130f to connect with a patient return electrode (not shown).

According to an exemplary embodiment of the present disclosure, connector interfaces, such as bipolar connector interfaces 1130a, 1130b, are configured to receive the connector interface 900 of the bipolar energy transmission cable 142. The bipolar connector interfaces 1130a, 1130b each include a pair of female flux transmission terminals 1132 spaced laterally apart from one another and configured to facilitate the transmission of electrical energy from the flux generator 204. The bipolar connector interfaces 1130a, 1130b also include a data signal transmission terminal 1136 spaced vertically from the female flux transmission terminals 1132. In the exemplary embodiment of FIG. 11, the data signal transmission terminal 1136 is an electrical finger or recess configured to make contact with the terminal 904 of FIG. 9A. In an exemplary embodiment, the terminal 1136 may be a copper terminal. The data signal transmission terminal 1136 can receive identification data from, e.g., the bipolar electrosurgical instrument 1202 (shown in FIG. 12) to provide it to a data signal processor, e.g., data interface module 208. Specifically, the data signal transmission terminal 1136 is in data signal communication with the data signal processor.

The connector interface 900 of the bipolar energy transmission cable 142 is configured to mate with the bipolar flux source connector interfaces 1130a, 1130b. The male flux transmission terminals 902 are configured to be received in the female flux transmission terminals 1132. When electrical energy is supplied through one of the bipolar connector interfaces 1130a, 1130b, the energy is transmitted from a flux generator via the female flux transmission terminals 1132 to the energy transmission line of the bipolar energy transmission cable 142. The data signal transmission terminal 904 of the bipolar energy transmission cable 142 is configured to interface with the corresponding data signal transmission terminal 1136 at one of the bipolar flux source connector interfaces 1130a, 1130b. Identification data of the electrosurgical instrument 1202 is provided through the data signal transmission terminal 904 and data transmission line of the cable 142 to the corresponding data signal transmission terminal 1136, which provides the identification data to a data signal processor, e.g., data interface module 208. The data signal processor may be provided at the ESU 1102.

In addition to being configured to be received at a flux supply source connector interface 1130a, 1130b of the present disclosure, the connector interface 900 of the bipolar energy transmission cable 142 is configured to mate with a standard flux supply source connector interface of, for example, a conventional electrosurgical unit (not shown). In particular, the flux transmission terminals 902 are also configured to mate with, and supply flux from, corresponding flux transmission terminals of a conventional flux supply source. Thus, users of the bipolar transmission cable 142 of FIGS. 9A-9C are not required to use an extra cable when converting between the ESU 1102 (including the flux source connector interface 1130, 1130b) of the present disclosure and a conventional flux supply source connector interface that does not include, for example, a bipolar connector interface with a data terminal.

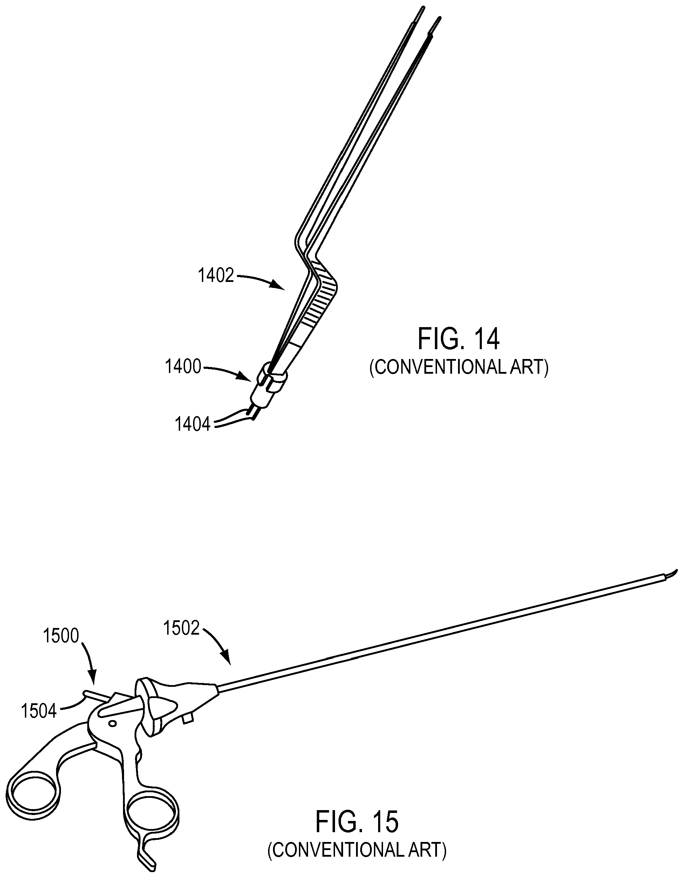

Turning now to FIGS. 9B and 9C, connector interfaces 910, 920 at the opposite end of connector interface 900 and for connection with a bipolar surgical instrument 1202 (the housing portion of the instrument that interfaces with an actuation interface assembly at the patient side cart being shown in FIG. 12) are shown. The connector interfaces 910, 920 include a pair of flux transmission terminals 912, 922, which are female connectors in the exemplary embodiment of FIGS. 9B and 9C, spaced apart from one another. The connector interfaces 910, 920 are configured to engage with a corresponding instrument connector interface, such as a conventional connector interface 1400 shown in FIG. 14, and also are configured to engage with the instrument connector interface 1200 of the bipolar electrosurgical instrument 1202 according to an exemplary embodiment, shown in FIG. 12. The connector interfaces 910, 920 are configured to transmit flux to the instrument, e.g., electrosurgical instrument 1202, through the bipolar energy transmission cable 142. Each pair of the flux transmission terminals 912, 922 is configured to engage with a corresponding pair of male flux transmission terminals 1404, shown in FIG. 14, at a connector interface 1400 of a conventional bipolar electrosurgical instrument 1402 and provide electrical energy thereto. As will be discussed in more detail below, each set of the flux transmission terminals 912, 922 is also configured to engage with a corresponding set of male flux transmission terminals 1204, shown in FIG. 12, at an instrument connector interface 1200 on a bipolar electrosurgical instrument 1202 in accordance with an exemplary embodiment of the present disclosure.

In addition to the terminals 912, 922, the connector interfaces 910, 920 includes a data signal transmission terminal 914, 924 which, in the exemplary embodiments of FIGS. 9B and 9C, is a female connector, spaced vertically apart from the pair of flux transmission terminals 912, 922. The data signal transmission terminal 914, 924, as will be described in more detail below, provides identification data signals from, e.g., the electrosurgical instrument 1202 to a data signal processor, e.g., data interface module 208, 309. Specifically, the data signal transmission terminal 914, 924 is an interface of a data signal transmission line extending through the bipolar energy transmission cable 142. The bipolar energy transmission cable 142 interfaces with, for example, a flux delivery device, e.g., the bipolar electrosurgical instrument 1202, that is provided with identification data. The identification data is provided from the bipolar electrosurgical instrument 1202 through the data signal transmission line via the data signal transmission terminals 914, 924. The data signal transmission terminals 914, 924 are configured to engage with a corresponding data signal transmission terminal 1206 of the bipolar electrosurgical instrument 1202, shown in FIG. 12, in order to receive the identification data from the bipolar electrosurgical instrument 1202. The connector interfaces 910, 920 also are configured to engage with a conventional instrument connector interface, such as connector interface 1400 that does not include a data transmission capability, as shown in FIG. 14 for example. However, the data signal transmission terminals 914, 924 does not provide identification data from a conventional bipolar electrosurgical instrument, such as instrument 1402, as the conventional bipolar electrosurgical instrument does not transmit identification data or include a data signal transmission terminal at the connector interface 1400 but rather only includes the two bipolar energy terminals 1404.

Referring to FIG. 12, in an exemplary embodiment, the flux delivery instrument connector interface 1200 can be a recessed interface. The connector interface 1200 includes a pair of flux transmission terminals 1204, which are male connectors in the exemplary embodiment of FIG. 12, spaced apart from one another. The flux transmission terminals 1204 are configured to transmit flux from the bipolar energy transmission cable 142 to the bipolar electrosurgical instrument 1202. The connector interface 1200 additionally includes a data signal transmission terminal 1206, which is a male connector in the exemplary embodiment of FIG. 12, spaced apart from the pair of flux transmission terminals 1204. The data signal transmission terminal 1206 is in communication with an electronic chip at the bipolar electrosurgical instrument 1202. The data signal transmission terminal 1206 is configured to receive identification information of the bipolar electrosurgical instrument 1202 and transmit the identification information through the data transmission terminals 914, 924 of the bipolar energy transmission cable 142.

In the exemplary embodiment of FIG. 12, the recessed connector interface 1200 is designed to receive the connector interfaces 910, 920 shown in the embodiment of FIG. 9B and 9C, the shape of the connector interfaces 910, 920 (e.g., having a larger, rounded shape around the flux transmission terminals 922 and a smaller, square shape at the data signal transmission terminal 924 corresponds with the shape of the recessed connector interface 1200. The corresponding shapes of portions of the connector interfaces 910, 920, and 1200 facilitate correct orientation of the connector interfaces 910, 920 of the bipolar energy transmission cable 142 in relation to the connector interface 1200 of the bipolar electrosurgical instrument 1202.

In accordance with another exemplary embodiment, the flux transmission conduit may be a monopolar energy transmission cable, such as monopolar energy transmission cable 144. FIG. 10A shows a first connector interface 1000 configured to connect with flux source connector interfaces 1130c, 1330d (shown in FIG. 11), which in an exemplary embodiment may be provided at the ESU 1102. FIGS. 10B and 10C show different configurations of another connector interface 1010, 1020 of the monopolar energy transmission cable 144 (shown in part in FIGS. 10B and 10C) configured to connect with a monopolar electrosurgical instrument 1302 at an instrument connector interface 1300 (the housing portion of the instrument that interfaces with an actuation interface assembly at the patient side cart being shown in FIG. 13).

As shown in FIG. 10A, the connector interface 1000 includes a single male flux transmission terminal 1002, configured to be placed in electrical energy contact with a connector interface at a flux supply source. The connector interface 1000 is configured to engage with a corresponding flux source connector interface to transmit flux from the flux supply source through the monopolar cable 144. In addition to the terminal 1002, the connector interface 1000 includes a data signal transmission terminal 1004, which may be, for example, an electrical contact ring, such as copper ring. The data signal transmission terminal 1004 transmits identification data from, e.g., the electrosurgical instrument 1302 (shown in FIG. 13) to a data signal processor, e.g., data interface module 208 , 309, at the flux supply source. The data signal transmission terminal 1004 is an interface of a data signal transmission line extending through the monopolar energy transmission cable 144. When the monopolar energy transmission cable 144 interfaces with, for example, an electrosurgical instrument that is provided with identification data, the identification data is provided from the electrosurgical instrument through the data signal transmission line to the data signal transmission terminal 1004.

Referring back to FIG. 11, in an exemplary embodiment, flux supply source connector interfaces, such as monopolar connector interfaces 1130c, 1130d, are configured to connect to the monopolar energy transmission cable 144. The monopolar connector interfaces 1130c, 1130d each include a single female flux transmission terminal 1134 configured to receive the male terminal 1002 of FIG. 10C to enable the monopolar energy cable 144 to transmit electrical energy from the flux supply source 1102. The monopolar connector interfaces 1130c, 1130d also include a data signal transmission terminal 1138 spaced vertically from the female flux transmission terminal 1134. In the exemplary embodiment of FIG. 11, the data signal transmission terminal 1138 is an electrical finger or recess configured to mate with the data transmission terminal 1004 of the monopolar energy transmission cable connector interface 1000. In an exemplary embodiment, the data signal transmission terminal 1004 may be copper. The data signal transmission terminal 1138 enables the transmission of identification data from, e.g., the monopolar electrosurgical instrument 1302 (shown in FIG. 13) to a data signal processor, e.g., data interface module 208, 309. Specifically, the data signal transmission terminal 1138 is in data signal communication with the data signal processor.

When electrical energy is supplied through one of the monopolar connector interfaces 1130c, 1130d, the energy is transmitted from the flux generator via the female flux transmission terminal 1134 through the energy transmission line of the monopolar energy transmission cable 144. Also, identification data of the electrosurgical instrument 1302 is provided through the data signal transmission terminal 1004 and data transmission line of the conduit 144 to the corresponding flux supply source data signal transmission terminal 1138, which provides the identification data to a data signal processor, e.g., data interface module 208. The data signal processor may be provided at the ESU 1102.

In addition to being configured to be received at a flux supply source connector interface 1130c, 1130d of the present disclosure, the connector interface 1000 of the monopolar energy transmission cable 144 is configured to mate with a conventional flux source connector interface of, for example, a conventional ESU (not shown). In particular, the flux transmission terminals 1002 also are configured to mate with, and supply flux from, corresponding flux transmission terminals of a conventional flux supply source. Thus, users of the monopolar energy transmission cable 144 are not required to use an extra cable when converting between a flux source in accordance with exemplary embodiments of the present disclosure (e.g., the ESU 1102 including the flux source connector interface 1130c, 1130d) and a conventional flux source connector interface that does not include, for example, a monopolar connector interface with a data terminal.

Turning now to FIGS. 10B and 10C, connector interfaces 1010, 1020 at the opposite end of connector interface 1000 of the monopolar energy transmission cable 144 are shown. The connector interfaces 1010, 1020 are configured to engage with a corresponding monopolar instrument connector interface, e.g., connector interface 1300 of a monopolar electrosurgical instrument 1302, shown in FIG. 13, and also with a conventional connector interface 1500 of a conventional monopolar instrument, as shown in FIG. 15. The connector interfaces 1010, 1020 include two flux transmission terminals 1012 and 1014, and 1022 and 1024, respectively, which are female connectors in the exemplary embodiment of FIGS. 10B and 10C, spaced apart from one another. Each of the flux transmission terminals 1012 and 1022 are configured to engage with a corresponding flux transmission terminal 1504, shown in FIG. 15, of a conventional instrument connector interface 1500 on a conventional monopolar electrosurgical instrument 1502 and provide electrical energy thereto.

The flux transmission terminals 1014, 1024 are sized smaller than the flux transmission terminals 1012, 1022, which correspond with conventional flux transmission terminals of a conventional instrument connector interface. The flux transmission terminals 1014, 1024 are configured to mate with a corresponding flux transmission terminal 1304 at the connector interface 1300 of the monopolar electrosurgical instrument 1302, as shown in an exemplary embodiment in FIG. 13. In an exemplary embodiment, the connector interface 1020 of FIG. 10C is configured to engage with the instrument connector interface 1300 shown in FIG. 13 to transmit flux to a monopolar electrosurgical instrument 1302 through the monopolar energy transmission cable 144.

In addition to the terminals 1012 and 1014, and 1022 and 1024, the connector interfaces 1010, 1020 include at least one data signal transmission terminal, 1016a and 1016b, or 1026. In the exemplary embodiments of FIGS. 10B and 10C, the at least one data signal transmission terminal 1016a, 1016b, or 1026 is a female connector, spaced apart from the flux transmission terminals 1012 and 1014, and 1022 and 1024, respectively. The at least one data signal transmission terminal 1016a, 1016b, or 1026, as will be described in more detail below, facilitates providing identification data from, e.g., the monopolar electrosurgical instrument 1202 to a data signal processor, e.g., data interface module 208. Specifically, the data signal transmission terminal 1016a, 1016b, or 1026 is an interface of a data signal transmission line extending along the monopolar energy transmission cable 144. When the monopolar energy transmission cable 144 interfaces with, for example, the monopolar electrosurgical instrument 1302 is provided with identification data, the identification data is provided from the monopolar electrosurgical instrument 1302 through the data signal transmission line via the data signal transmission terminal 1016a, 1016b, or 1026.

Referring to FIG. 13, in an exemplary embodiment, the flux delivery device connector interface 1300 is a recessed interface provided at a flux delivery device, e.g., monopolar electrosurgical instrument 1302. The connector interface 1300 includes the single flux transmission terminal 1304, which is a male connector in the exemplary embodiment of FIG. 13. The flux transmission terminal 1304 is configured to supply flux from a monopolar energy transmission cable 144 to the monopolar electrosurgical instrument 1302. The connector interface 1300 additionally includes the data signal transmission terminal 1306, which is a male connector in the exemplary embodiment of FIG. 13, spaced apart from the flux transmission terminal 1304. The data signal transmission terminal 1306 is in communication with an electronic chip at the monopolar electrosurgical instrument 1302. The data signal transmission terminal 1306 is configured to receive identification information of the monopolar electrosurgical instrument 1302 and transmit the identification information through the monopolar energy transmission cable 144.

The female flux transmission terminal 1014, 1024 is configured to mate with the male flux transmission terminal 1304. When electrical energy is supplied through the monopolar energy transmission cable 144, the energy is transmitted via the female flux transmission terminal 1014, 1024 through the energy transmission line within the monopolar energy transmission cable 144 to the male flux transmission terminal 1304 into the monopolar instrument 1302. The data signal transmission terminal(s) 1016a and 1016b, or 1026 of the monopolar energy transmission cable 144 is configured to mate with a corresponding data signal transmission terminal(s), e.g., data signal transmission terminal 1306 at the connector interface 1300 of the monopolar electrosurgical instrument 1302 (mating data signal transmission terminals for 1016a, 1016b not shown). Identification data of the electrosurgical instrument 1302 is provided through the data signal transmission terminal 1306 to the corresponding cable data signal transmission terminal 1016a and 1016b, or 1026 and transmitted through the data signal transmission line of the monopolar energy transmission cable 144.

In the exemplary embodiment of FIG. 13, the recessed connector interface 1300 is designed to receive the connector interface 1020 shown in the embodiment of FIG. 10C, where the shape of the connector interface 1020 (e.g., having a larger, rounded shape in the middle of the interface 1020, a smaller, rounded shape at one end of the interface 1020 and a smaller, square shape at another end of the interface 1020) of the monopolar energy transmission cable 144 corresponds with the shape of the recessed connector interface 1300. The corresponding shapes of portions of the connector interfaces 1020, 1300 facilitate correct orientation of the connector interface 1020 of the monopolar energy transmission cable 144 in relation to the connector interface 1300 of the monopolar electrosurgical instrument 1302.

One of ordinary skill in the art would recognize that any of the female terminals of the energy transmission cables, flux source connector interfaces, and instrument connector interfaces can be male terminals and vice versa. Those having ordinary skill in the art would appreciate other modifications that may be needed to the various connectors and receptacles in order to accommodate a modification of the terminal types.

In various exemplary embodiments, the flux transmission terminals and the data signal transmission terminals are sized and arranged such that they are able to mate only to corresponding respective flux transmission terminals and the data signal transmission terminals.

One of ordinary skill in the art would recognize that various other designs may be chosen for the connection of data signal transmission terminals between connector interfaces and flux transmission terminals between connector interfaces, as long as a mating or connecting relationship is formed between the appropriate terminals. Other design configurations in accordance with various exemplary embodiments can be selected, for example, by satisfying a set of design criteria set forth below in Table 1. Table 1 illustrates exemplary connecting and data transmission conditions of energy transmission cables (for both bipolar and monopolar energy) in accordance with exemplary embodiments of the present disclosure, to both conventional energy supply sources and energy supply sources of the present disclosure and to both conventional (e.g., handheld laparoscopic) electrosurgical instruments and electrosurgical instruments in accordance with the present disclosure (for both bipolar and monopolar instruments).

**Table 1:**

| | Instrument (e.g., mono-or bi-polar energy) in accordance with present disclosure | Conventional (e.g., mono-or bi-polar energy) instrument | Energy supply source (e.g., mono- or bipolar energy) in accordance with present disclosure | Conventional energy supply source (e.g., mono- or bipolar energy) |
|---|---|---|---|---|
| Cable (e.g., mono- or bipolar energy) in accordance with present disclosure | Connects; Identification data transmitted | Connects; no identification data transmitted | Connects; identification data received | Connects; no identification data received |
| Conventional (e.g., mono-or bi-polar energy) cable | Does not connect | Connects; no identification data transmitted | Connects; no identification data received | Connects; no identification data received |

In addition to the design criteria above, in accordance with various exemplary embodiments, the bipolar energy transmission cable 142 and the monopolar energy transmission cable 144 are designed so that the energy transmission cables 142, 144 are not interchangeable. In other words, the connector interfaces at each end of the respective cables 142, 144 are configured such that none of the connector interfaces 900, 910, or 920 at the ends of the bipolar energy transmission cable 142 can be connected with either the monopolar electrosurgical instrument connector interface 1300 or the monopolar flux supply source connector interfaces 1130c, 1130d, respectively. Similarly, none of the connector interfaces 1000, 1010, or 1020 at the ends of the monopolar energy transmission cable 144 can be connected with either the bipolar electrosurgical instrument connector interface 1200 or the biopolar flux supply source connector interfaces 1130a, 1130b, respectively. In this way, inadvertently routing monopolar energy to the bipolar electrosurgical instrument 1202 or bipolar energy to the monopolar electrosurgical instrument 1302 can be avoided. The overall connector interface shape, as well as the positioning and number of the flux transmission terminals and the data signal transmission terminals at both ends of each of the bipolar and monopolar energy transmission cables 142, 144 can be selected to uniquely differentiate the connector interfaces of the bipolar energy transmission cable 142 from the corresponding connector interfaces of the monopolar energy transmission cable 144. The connector interfaces 1200 and 1300 and the connector interfaces 1130a, 1130b and 1130c, 1130d also may have such unique and differentiating configurations so as to be able to mate only with the corresponding bipolar or monopolar cable.

Further, in accordance with various exemplary embodiments, the connector interfaces of the bipolar electrosurgical instrument 1202 or the monopolar electrosurgical instrument 1302 of the present disclosure are configured to prevent mating engagement with the connector interfaces of conventional bipolar and monopolar energy transmission cables (not shown and configured to engage with conventional bipolar or monopolar electrosurgical instruments). For example, referring to the exemplary embodiments of FIGS. 12 and 13, the shape and recessed configuration of the connector interfaces 1200, 1300 of the bipolar electrosurgical instrument 1202 and the monopolar electrosurgical instrument 1302, respectively, can be selected to prevent mating engagement of connector interfaces of conventional bipolar and monopolar energy transmission cables.

Moreover, in accordance with various exemplary embodiments, the configuration of the connector interfaces 910, 920, and 1010, 1020 of the bipolar energy transmission cable 142 and the monopolar energy transmission cable 144 are configured to mate to surgical instruments, such as electrosurgical instruments 1202, 1302 that are able to transmit data and provide the unique instrument identification information via the transmission cables 142, 144. However, the connector interfaces 910, 920, 1010, 1020 also are configured to mate to conventional electrosurgical instruments, such as those having conventional banana plug connector interface, that do not have data signal transmission capabilities via an energy transmission cable (e.g., via the energy connector interfaces of the conventional surgical instruments). In addition, the connector interfaces 900 and 1000 of the bipolar energy transmission cable 142 and the monopolar energy transmission cable 144 are configured to be mated to flux supply source connector interfaces 1130a, 1130b, and 1130c, 1130d that can receive and read data signals transmitted through the cables 142, 144. The connector interfaces 900, 1000 also are configured to mate to conventional flux source connector interfaces that include corresponding flux transmission terminals but do not include data signal terminals. This permits the use of energy transmission cables in accordance with the present disclosure with conventional flux supply sources (e.g., conventional electrosurgical generation units), for example, to transmit energy but that do not receive and read data signals through the energy transmission cable.

In accordance with various exemplary embodiments, the overall look and shape of the connector interfaces 910, 920, 1010, 1020 of the flux transmission conduits, e.g., cables 142, 144, are selected so as to be aesthetically appealing. The unique look and shape also allows users to readily recognize the transmission conduits, e.g., cables 142, 144, of the present disclosure from conventional flux transmission cables (e.g., electrosurgical energy transmission conduits) and from each other (e.g., bipolar versus monopolar). Similarly, the overall look and shape of the connector interfaces 1200, 1300 of the electrosurgical instruments 1202, 1302 also are selected so as to be aesthetically appealing and readily distinguishable from conventional instruments and each other. The unique shape also allows users to easily determine the appropriate flux transmission conduit that can be matingly engaged.

### Controlling Supply of Flux to Instruments

Referring now to the schematic illustration of FIG. 7, a portion of an exemplary embodiment of a manipulator arm 7110 of a patient side cart with two surgical instruments 7102A, 7102B in an installed position is shown. The schematic illustration of FIG. 7 depicts only two surgical instruments for simplicity, but more than two surgical instruments may be received in an installed position at a patient side cart as those having ordinary skill in the art are familiar with. Each surgical instrument 7102A, 7102B includes an instrument shaft 7103A, 7103B that at a distal end has a moveable end effector (not shown in FIG. 2), and may or may not include a wrist mechanism (also not shown in FIG. 2) to control the movement of the end effector. In the exemplary embodiment of FIG. 7, the distal end portions of the surgical instruments 7102A, 7102B are received through a single port structure 7104 to be introduced into the patient. Other configurations of patient side carts that can be used in conjunction with the exemplary embodiments described herein can use several individual manipulator arms on which each surgical instrument is installed, such as, for example, the patient side cart 110 depicted in the exemplary embodiment of FIG. 1. Transmission housings 7203A, 7203B disposed at a proximal end of each shaft 7103A, 7103B may connect through a sterile adaptor 7206A, 7206B with actuation interface assemblies 7706A, 7706B.

In various exemplary embodiments, at least one of the surgical instruments 7102A is in communication via a flux transmission conduit 7106 with a flux source 7108 such that when the surgeon (e.g., at a surgeon side console 120 in the exemplary embodiment of FIG. 1) provides a flux input command during a surgical procedure, a controller (e.g., at the control cart 130) sends a signal to the flux source 7108 and the surgical instrument 7102A is activated by flux supplied from the flux source 7108 through the flux transmission conduit 7106. In various exemplary embodiments, the surgical instruments 7102A, 7102B are electrosurgical instruments, the flux transmission conduit 200 is an electrical energy transmission cable, and the flux source 7108 is an electrical energy generator. Thus, when the surgeon (e.g., at a surgeon side console 120) inputs a command for electrical energy during a surgical procedure (e.g., a cautery command such as by pressing a pedal 124b, 124d of surgeon side console 120), a signal is sent via the controller (e.g., at control cart 130 or otherwise distributed across the various teleoperated surgical system components) to the electrical energy generator 7108. This in turn activates the electrosurgical instrument 7102A by electrical energy supplied from the electrical energy generator 7108 through the electrical connection cable 7106.

As discussed above, various exemplary embodiments of a teleoperated surgical system contemplate the ability to determine which of a plurality of surgical instruments that are in installed positions (e.g., transmission mechanisms and actuation interface assemblies engaged) at a patient side cart are in communication with a flux source by detecting the presence of an flux transmission conduit connected to a respective instrument. Such detection can be accomplished using various sensing mechanisms, various exemplary embodiments of which are described in further detail below.

Thus, with reference again to FIG. 7, a teleoperated surgical system in accordance with at least one exemplary embodiment, can determine that an electrosurgical instrument 7102A is in electrical communication with the electrical energy generator 7108 via the detection of the electrical energy transmission cable 7106 being connected to the electrosurgical instrument 7102A. Based on such detection, various control schemes can be implemented and/or information regarding the same can be provided as feedback, for example, to a surgeon.

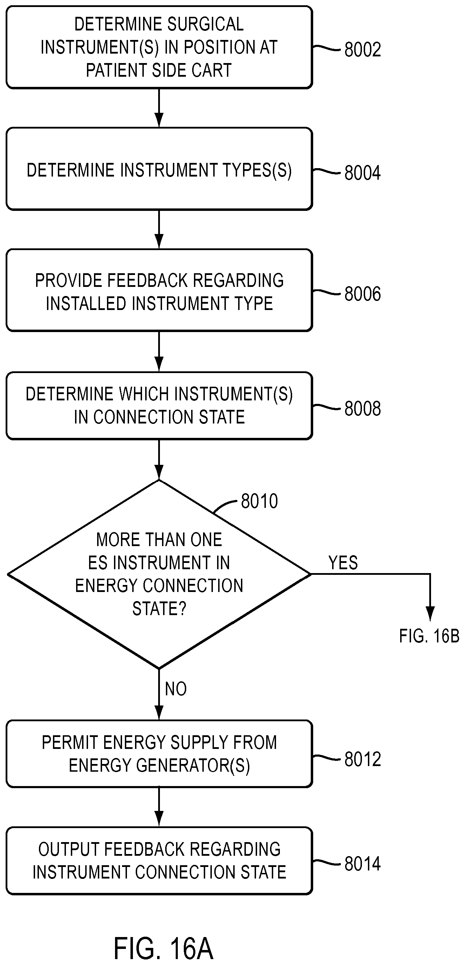

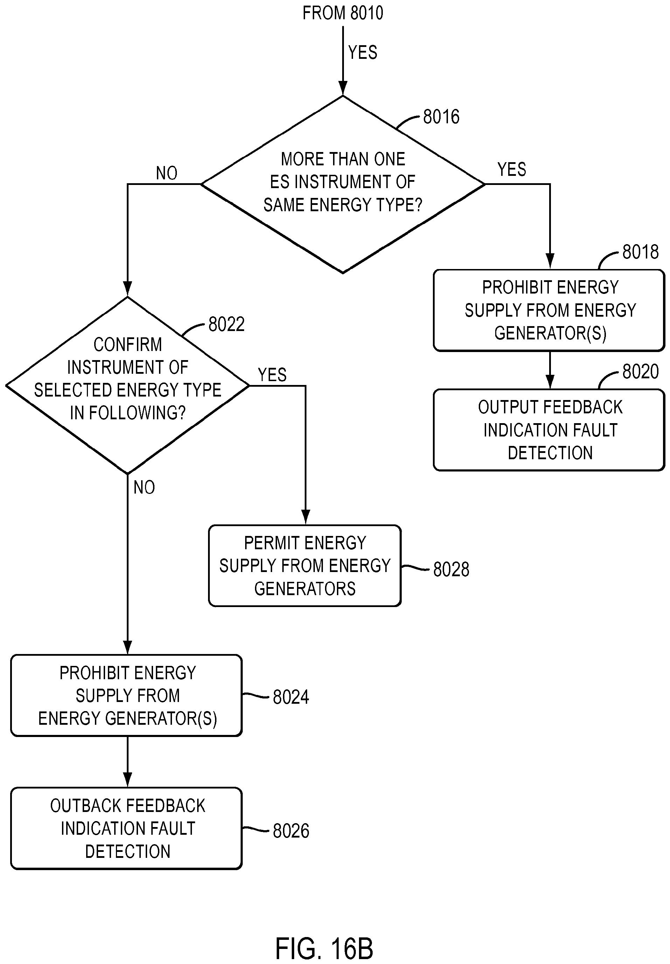

A flow diagram that depicts exemplary steps of a workflow in accordance with various exemplary embodiments is shown in FIGS. 16A and 16B. It should be understood that each of the steps depicted may not be required in any particular embodiment and some of the steps depicted may occur in other orders and/or more than once than those illustrated.

For ease of understanding, the exemplary workflow of FIGS. 16A and 16B is described below with reference to implementation on various components of the exemplary embodiments of FIGS. 1 and 7. However, those having ordinary skill in the art will appreciate that such description with reference to FIGS. 1 and 7 is not intended to be limiting, and the workflow of FIGS. 16A and 16B can be implemented on a variety of teleoperated surgical systems. Further, the exemplary workflows are described in terms of electrical energy being the type of flux delivered to the surgical instrument, but as above, those skilled in the art will understand that other flux types can be delivered and are contemplated as within the scope of the present disclosure.

With reference now to the FIGS. 16A and 16B, as well as the system components in the exemplary embodiment of FIG. 7, in various exemplary embodiments, at 8002 in FIG. 16A, a determination is made that one or more surgical instruments 7102A are installed at a patient side cart. This determination can be made in various ways, including but not limited to for example, providing one or more memory structure reader devices (shown schematically at 7112A, 7112B in FIG. 7) at the patient side cart, for example, in proximity to or associated with each actuation interface assembly 7706A, 7706B. The instrument reader devices can read corresponding memory storage structures provided on the surgical instruments and containing various information about the surgical instruments, such as for example, instrument type, number of uses, etc., as those skilled in the art are familiar with.

Exemplary instrument reader devices can include, but are not limited to, for example, RFID sensors that read a unique RFID tag that is disposed on each surgical instrument; a memory chip (e.g., a SRAM or EEPROM memory chip) reader that can interface with and receive data from a unique memory chip provided on each surgical instrument; a barcode reader; and/or a magnetic medium reader (e.g., magnetic strip reader). Those having ordinary skill in the art will appreciate other types of reader devices can be used that have the ability to read stored information from a readable or readable and writable memory storage structure associated with the instrument. Such memory storage structure reader devices can be proximity-based in that their ability to read the unique information with which an instrument memory storage device is programmed relies on the instrument being in close range, e.g., coupled or engaged, with a respective actuation interface assembly 7706A.

In addition to detecting instrument presence, a workflow in accordance with an exemplary embodiment can determine the instrument type of an instrument installed at an actuation interface assembly 7706A. For example, the instrument reader devices associated with each actuation interface assembly 7706A, 7706B e.g., RFID and/or memory chip readers, can determine instrument type and other information regarding an instrument based on the programmed information on the unique memory storage structure (e.g., RFID tag and/or memory chip). With regard to instrument type, the unique identifier information associated with a respective surgical instrument can include whether or not it is a surgical instrument configured for energy delivery (e.g., an electrosurgical instrument) and what type of energy it is configured to deliver (e.g., bipolar, monopolar, mixed mode, etc.).

Other types of proximity-based sensing devices also may be used, in lieu of or in addition to, for example, the RFID and/or memory chip readers, to detect that a surgical instrument 7102A is in an installed position at an actuation interface assembly 7706A. For example, and as described in more detail below, a proximity-based sensing device can be disposed in the interface actuation assemblies 7706A, 7706B and configured to sense when a transmission housing 7203A of a surgical instrument 7102A is engaged therewith so as to confirm a surgical instrument is in an accurate and installed position at a respective actuation interface assembly. In this way, it is possible to confirm instrument installation at a patient side cart.

At step 8004, the information obtained by the instrument reader and/or instrument installation sensing devices can be sent as signal information to the controller, as those of ordinary skill in the art would have familiarity with, and the controller can thus determine the instrument types that are installed at the respective actuation interface assemblies at the patient side cart.

At 8006, in at least one exemplary embodiment, the controller can output feedback regarding at least the instrument type, and optionally the confirmed installation, of each respective instrument 7102A, 7102B that is installed at each respective actuation interface assembly 7203A, 7203B. By way of example, such information may be provided on display 132 of control cart 130 to enable a surgeon at the surgeon side console 120 to know which input devices (e.g., 122, 124a-124d) will operate which instruments 102, 7102A, 7102B upon an input command being provided at one of those input devices, with the surgeon also being provided with information regarding which input devices are mapped to control which instrument arms.

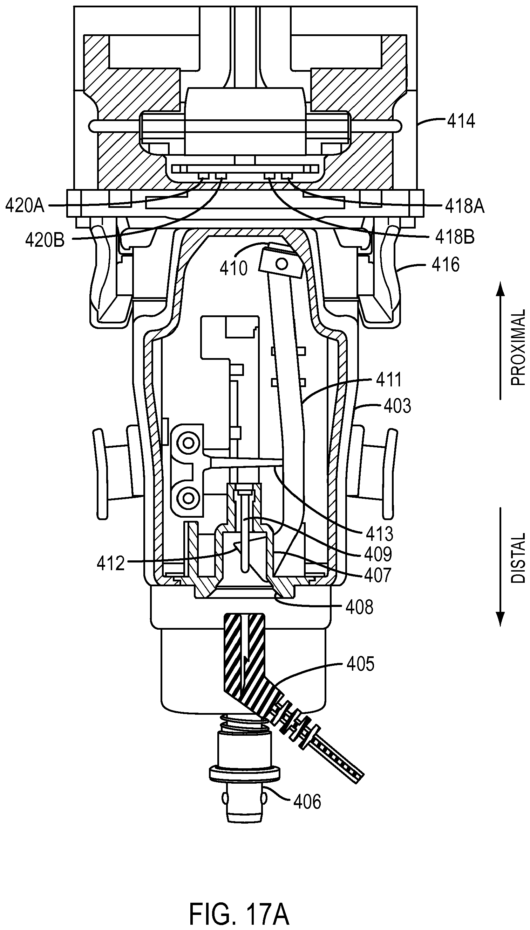

At 8008, if a plurality of electrosurgical instruments are determined to be installed at a patient side cart, a determination can be made at the controller which of the electrosurgical instruments is in an energy connection state by detecting the electrosurgical instruments that have an electrical energy transmission cable engaged therewith. Various exemplary embodiments are described in more detail below with reference to FIGS. 17A-24 of non-limiting exemplary embodiments of "cable presence" sensing devices that can be used to provide a signal to the controller indicating that a respective electrosurgical instrument has an energy transmission cable in engagement therewith. Based on the signals indicative of whether and which electrosurgical instrument(s) are in an energy connection state, the controller can control the energy delivery to the various installed electrosurgical instruments.

At 8012 the controller can control the energy generator (e.g., 7108 in the exemplary embodiment of FIG. 7 or 202 in the exemplary embodiment of FIG. 1 or others not shown) to which an electrosurgical instrument 7102A is in electrical communication (e.g., send a signal closing a relay at the energy generator) to deliver energy upon receipt of an energy input command from the surgeon side console 120. In various exemplary embodiments, it is contemplated that an electrosurgical instrument can be configured to deliver differing types of energy, such as, for example, bipolar and monopolar, respectively. In such case it is contemplated that in at least one exemplary embodiment differing energy generators may be provided, with the energy transmission cable connecting the instrument to one of the generators depending on the energy type desired for the surgical procedure being performed. Again, the controller can send a signal to the energy generator requested based on an energy input command at the surgeon side console 120. In various exemplary embodiments, one of the pedals 124a-124d can be associated with providing bipolar energy while another is associated with providing monopolar energy, and respective energy generators can be utilized to supply one of those types of energies. Other configurations also are contemplated and the input devices at the surgeon side console can be mapped with one or more energy generators in numerous ways within the controller, and such mapping information shared with the surgeon, without departing from the scope of the present disclosure.

For example, a left bank of pedals can be mapped to control energy delivery to instruments that are being operated by the left hand gripping mechanism 2020 of the surgeon side console, while a right bank of pedals can be mapped to control energy delivery to instruments that are being operated by the right hand gripping mechanism 2020. For this and other positional mapping that can be utilized, reference is made to U.S. Patent Application No. 14/028,006, publication no. US 2014-0081455 A1, filed September 16, 2013 (for "METHODS AND SYSTEMS FOR ASSIGNING INPUT DEVICES TO TELEOPERATED SUGICAL INSTRUMENT FUNCTIONS"). Each bank could also have differing energy types associated with it; for example one pedal of each bank could be mapped to supply energy from a monopolar energy source and another pedal of each bank could be mapped to supply energy from a bipolar energy source. Those having ordinary skill in the art would understand how to make appropriate modifications based on the present disclosure to accommodate various formats for mapping energy input devices at the surgeon side console with energy delivery at the patient side cart.

At step 8010, if it is determined that there is not more than one electrosurgical instrument that is in an energy connection state, then the controller at 8014 may output feedback indicating which instrument is in the energy connection state. This can permit the surgeon to know that if a given energy input command is provided at the surgeon side console, for example, via depression of a pedal 124a-124d that is configured to provide an energy input command to the controller, which of the installed instruments that are electrosurgical instruments will be electrically activated, for example, to perform a cautery procedure on the patient.

If at 8010 the controller determines that more than one of the installed electrosurgical instruments is in an energy connection state, then the controller in an exemplary embodiment can implement a control scheme to prohibit energy delivery from one or more energy generators in cases where a potential ambiguity exists as to which of the instruments in a connection state would be electrically activated.

For example, with reference now to FIG. 16B, the controller at 8016 can determine, based on the instrument type information determined at 8004, whether or not more than one of the instruments determined to be in the connection state are the same energy type. For example, if it is determined that two or more electrosurgical instruments that are in an energy connection state are the same energy type (e.g., two bipolar instruments or two monopolar instruments), then, as shown at 8018 of the flow diagram of FIG. 16B, the controller in an exemplary embodiment prohibits the delivery of energy from any energy generators of the system due to the ambiguity of which instrument will be electrically activated upon an energy input command (which may be, e.g., an input for a particular energy type mapped to an input device such as, for example, a pedal 124a-124d at the surgeon side console 120) associated with more than one of the electrosurgical instruments that are in the energy connection state. By way of example, if at 8016 a determination is made that two or more surgical instruments that are in an energy connection state are bipolar energy type instruments, then, even upon receiving an energy input command from the surgeon side console, the controller will not transmit the requisite signal to the energy generator(s) in the system to deliver energy.

In an exemplary embodiment, in the case where one or more of the surgical instruments that are in an energy connection state is a mixed mode instrument, the controller can determine to which energy generator (e.g., bipolar or monopolar) the instrument is in communication with based on detecting which of differing energy transmission cable types (e.g., bipolar or monopolar) is in engagement with the instrument. For example, as will be better understood from the description of the exemplary embodiments of FIGS. 17A-24, a mixed mode surgical instrument may include two differing connection receptacles for bipolar energy supply and monopolar energy supply, respectively. Energy transmission cable sensing devices associated with the differing energy transmission cables may be used to detect when the differing cables are engaged with the surgical instrument. The energy type of the mixed mode instrument can then be effectively determined by detecting which energy transmission cable is engaged therewith. Based on this detection, if it is determined at 8016 that the mixed mode surgical instrument is effectively of the same energy type as another instrument, including another mixed mode instrument of the same effective energy type, then even upon receiving an energy input command from the surgeon side console, the controller at 8018 will not transmit the requisite signal to the energy generator(s) in the system to deliver energy.

At 8020 in FIG. 16B, the controller also can output fault detection feedback indicating that two or more electrosurgical instruments of the same energy type are detected to be in an energy connection state, leading to an ambiguous energy connection condition. For example, such feedback may be provided at the display 132 of control cart 130 and may include audible and/or visual feedback in various exemplary embodiments. In an exemplary embodiment, one or more of displays 132 can provide visual feedback indicating which instruments are in a connection state and/or indicating that the system is in a configuration wherein energy will not be delivered to the surgical instruments.

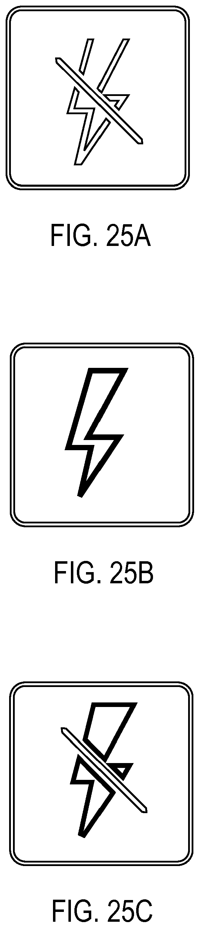

By way of non-limiting example only, FIGS. 25A-25C show exemplary icons that can be provided as visual feedback to indicate various states of an instrument for which an energy input command has been input at the surgeon side console. The icons in FIGS. 25A-25C can be displayed to show that an energy transmission cable is not connected (FIG. 25A), the surgical system is in a ready to activate energy state for a given surgical instrument (FIG. 25B), or the surgical system is in an ambiguous energy state (FIG. 25C) and thus energy activation will not be permitted. In an exemplary embodiment, the icons can be displayed on a display, such as display 132 of control cart 130, for example, and can be displayed in proximity to the name of the instrument they correspond to which also can be presented on the display. Those having ordinary skill in the art would appreciate numerous types of feedback, including other icons and/or other indications, could be presented to provide similar information to a user.

In various exemplary embodiments, the surgical system continuously monitors state changes, including, for example, monitoring of the energy connection states and types of the installed surgical instruments. Thus, upon a change in the energy connection state or otherwise rectifying any ambiguous condition determination, the control method and system returns to the previous workflow step to continue with the workflow, as described in further detail below.

If, on the other hand, at 8016 the controller determines that more than one electrosurgical instrument of the same type is not in an energy connection state, then at 8022 in FIG. 16B the controller can in accordance with an exemplary embodiment implement a control scheme whereby it confirms that the electrosurgical instrument of a particular energy type is in a current master-slave relationship with the surgeon side console, in other words "in following," as explained further below. For example, in some exemplary embodiments of teleoperated surgical systems encompassed within the scope of the present disclosure, there are fewer master input devices (e.g., two gripping mechanisms 122 at the surgeon side console 120) that are operable at a given time than there are actuation interface assemblies with installed surgical instruments (e.g., 3 or more at the patient side cart). In such a situation, the surgeon side console 120 is used to identify to the controller which of the plurality of actuation interface assemblies will be mapped and under control of a given master input devices. In the example above, the surgeon side console 120 identifies to the controller which of two actuation assemblies (e.g., 7203A, 7203B in the exemplary embodiment of FIG. 7) is mapped in a current master-slave relationship with the two gripping mechanisms 122. When an actuation interface assembly is in a master-slave relationship, the actuation interface assembly, and consequently the surgical instrument installed at that actuation interface assembly, are designated as "in following" and a signal indicative of that status is provided to the controller.

In the workflow of FIG. 16B, therefore, in various exemplary embodiments, as depicted at 8022, the controller upon receipt of an energy input command signal from the surgeon side console 120 confirms that the surgical instrument that the energy input command will activate is "in following," as described above. If at 8022 the designated instrument that would be activated by the energy input command is determined not to be "in following," at 8024 the controller prohibits energy from being delivered by the energy generator associated with that energy input command. For example, the controller will not send a signal closing a relay or otherwise providing input to the energy generator to supply energy to the instrument. Prohibiting energy delivery in this situation can enhance safety in the event that an instrument that is not "in following" is removed from the patient or at a position that the surgeon is not aware of due to the lack of a master-slave control over the instrument.

The controller also can output fault detection feedback signal at 8026 indicating that the instrument for which an energy input command was requested at the surgeon side console 120 is not "in following." For example, such feedback may be provided at the display 132 and may include audible and/or visual feedback in various exemplary embodiments.

If instead at 8022, the controller confirms that the surgical instrument that will be energy activated based on the energy input command at the surgeon side console 120 that is received as an energy input command signal at the controller is "in following," then at 8028 the controller will permit energy from an energy generator to be delivered to that instrument. For example, the controller will send a signal to the energy generator associated with the energy input command to close a relay and supply energy to the instrument. It should be noted that if two differing energy type instruments (e.g., bipolar and monopolar) are installed, in an energy connection state, and confirmed to be in following, then upon receipt of an energy input command for the particular energy type of those instruments from the surgeon side console, the controller can permit energy generator(s) to supply energy to both of those electrosurgical instruments.

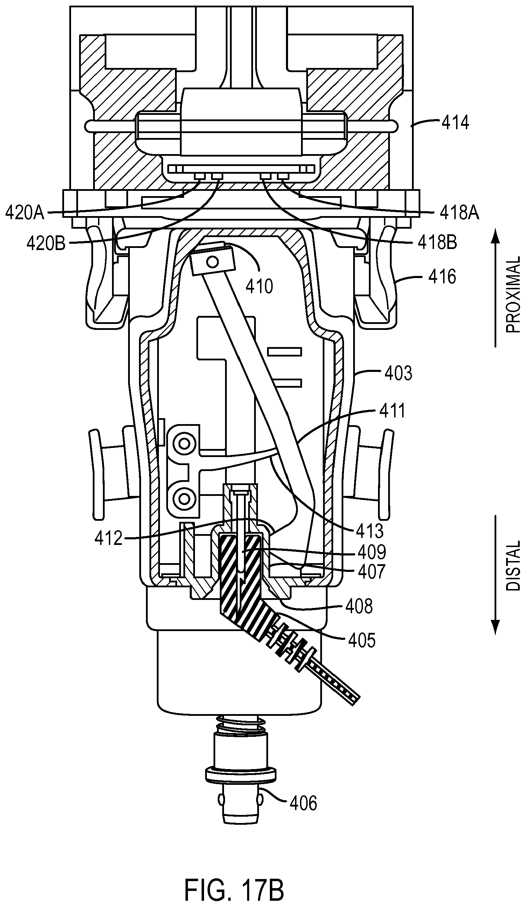

Referring now to FIGS. 17A and 17B, which depict cutaway views of a portion of an electrosurgical instrument housing in an installed position relative to an actuation interface assembly, one exemplary embodiment of a system for detecting both an installed position and energy connection state of the instrument is depicted. In the exemplary embodiment of FIGS. 17A and 17B, an electrosurgical instrument housing 403 is depicted in an installed position at an actuation interface assembly 414. The shaft and distal end portion of the surgical instrument that would connect to the housing at 406 is not depicted in FIGS. 17A and 17B. A connector receptacle 407 is disposed toward a distal end of the housing 403 and leads to an opening 408 that is accessible at an external surface of the housing 403. The opening 408 and connector receptacle 407 are sized to receive in mating engagement an energy transmission cable 405 (shown in partial view in FIGS. 17A and 17B), as those having ordinary skill in the art would be readily familiar. In a mating engaged state within the connector receptacle 407, the energy transmission cable 405 also is placed into contact with an energy contact member 409 to provide a closed energy circuit and permit energy (e.g., electrical current) to be transmitted from the cable 405 to the contact member 409, which ultimately is in energy communication with the distal end effector of the surgical instrument that delivers the energy to the patient. Although in FIGS. 17A and 17B, the cable 405 is depicted as having the female connection portion and the contact 409 is the male portion, those having ordinary skill in the art would appreciate that the male and female energy contact connector portions could be reversed.

In the exemplary embodiment of FIGS. 17A and 17B, the actuation interface assembly 414 is provided with a proximity-based sensing device that can be utilized to detect that the surgical instrument housing 403 is in an installed position. More specifically, one or more Hall sensors (e.g., one or more of Hall sensors 418A, 418B, 420A, and 420B as described in further detail below) is mounted in the actuation assembly interface 414. In turn, a magnet 410 can be disposed in the housing 403 at a location such that upon a correct engagement of the housing 403 with a sterile adapter 416 of the actuation interface assembly 414, which places the instrument housing 403 in an installed position relative to the interface actuation assembly 414 and thus a patient side cart, the magnet 410 is placed in sensing proximity to the one or more Hall sensors 418A, 418B, 420A, 420B. Detection of the magnetic field of the magnet 410 by the one or more Hall sensors 418A, 418B, 420A, 420B causes the Hall sensors to send a signal to the controller indicating the surgical instrument associated with the housing 403 is in an installed position with the actuation interface assembly 414 at the patient side cart. As will be better understood from the following description, it is desirable to provide a proximity-based sensing device and corresponding sensed component on the instrument (or vice versa) that enable detection of the instrument in the installed position regardless of other changes in instrument state, including the energy connection state, that may occur. Further, it may be desirable to provide a sensing device that is configured and arranged to be brought into sensing proximity with a sensed component upon accurate installation of the instrument at a manipulator arm, in order to be able to confirm such accuracy in installation of the instrument.

As described above with reference to the workflow of FIGS. 16A and 16B, the instrument, e.g., the instrument housing 403, also can be provided with a memory storage device (not shown), such as, for example, a RFID tag, EPROM, Flash EPROM, EEPROM, SRAM, etc., for storing instrument information and that is readable at a patient side cart when the housing 403 is installed at the actuation interface assembly 414.

In the exemplary embodiment of FIGS. 17A and 17B, the magnet 410 is disposed on the proximal end of a moveable arm 411 (e.g., a flexure arm), with the proximal and distal directions again being labeled in FIGS. 17A and 17B. More specifically, the arm 411 is mounted via a flexible hinge 413 within the housing 403. A cam feature 412 is provided at an end portion of the arm 411 opposite to the end at which the magnet 410 is mounted. In a configuration wherein the electrosurgical instrument is not in an energy connection state, as shown in FIG. 4A, with the cable 405 not received in mating engagement in the connector receptacle 407, the cam feature 412 projects through a slotted opening in the wall of the receptacle 407 and into the interior of the receptacle 407. In this state, the flexure arm 411 is in a first position (e.g., with the flexible hinge 413 in an unflexed configuration). In the position of the arm 411 in FIG. 17A, the magnet 410 is in sensing proximity to the one or more Hall sensors 418A, 418B.

In an energy connection state of the electrosurgical instrument, as illustrated in FIG. 17B, the mating engagement of the energy transmission cable 405 in the connector receptacle 407 places the energy transmission cable 405 into engagement with the cam feature 412. This engagement causes the cam feature 412 to move to the position shown in the FIG. 17B, which in turn causes the flexible hinge 413 to flex to pivot the arm 411 about the hinge 413 and move the magnet 410 to the position shown in FIG. 17B. In the position shown in FIG. 17B, the magnet 410 is in sensing proximity to one or more Hall sensors 420A, 420B in the actuation interface assembly 414. Upon sensing the magnet 410, the one or more Hall sensors 420A, 420B can send a signal to the controller 3004 indicating that the electrosurgical instrument associated with the housing 403 is in an energy connection state.

Although it is contemplated as within the scope of the present disclosure to use one Hall sensor 418 and one Hall sensor 420, providing Hall sensor pairs 418A, 418B and 420A, 420B can provide an ambiguous state detection feature that permits the controller to determine that the energy transmission cable is only partially received in the receptacle 407 but not entirely received therein (e.g., as shown in FIG. 17B) while also determining that the instrument is in an installed position. In this situation, in an exemplary embodiment, the controller can prohibit energy delivery to the instrument.

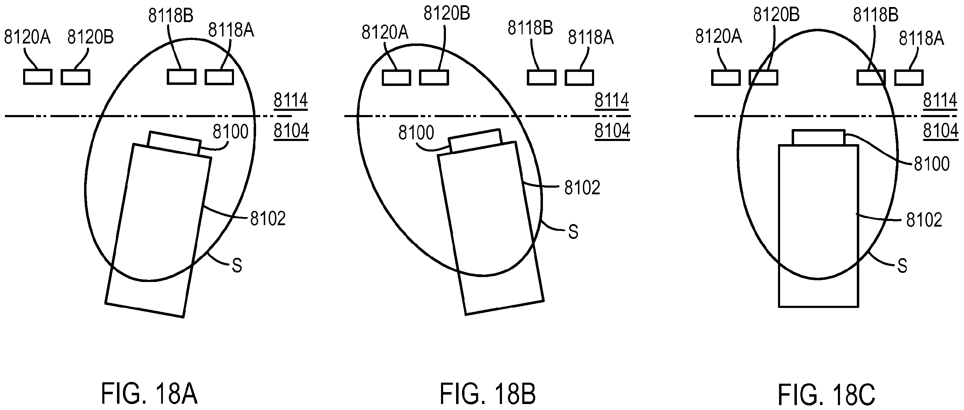

Referring now to FIGS. 18A-18C, an exemplary embodiment schematically depicting the use of two sets of Hall sensors in the actuation interface assembly to detect a magnet in the electrosurgical instrument housing is illustrated. In FIGS. 18A-18C, the dashed line depicts the interface between the electrosurgical instrument housing 8104 and the actuation interface assembly 8114. Similar to the description of FIGS. 17A and 17B, FIGS. 5A-5C depict a magnet 8100 disposed on the proximal end of a moveable arm 8102 and two sets of Hall sensors 8118A, 8118B and 8120A, 8120B disposed in the actuation interface assembly 8114. As shown in FIG. 18A, the arm 8102 and magnet 8100 are in a first position when the electrosurgical instrument is in an installed position at the actuation interface assembly 8114 and with the electrosurgical instrument not in an energy connection state (i.e., the energy transmission cable is not received in the connector receptacle as in the embodiment of FIG. 17A). In the first position, the magnet 8102 is in sensing proximity to both of the Hall sensors 8118A, 8118B as shown by the region S which indicates the magnetic flux range of the magnet 8100 having a strength sufficient to be detected by the Hall sensors 8118A, 8118B. In the first position, again as depicted by the region S, the Hall sensors 8120A, 8120B are not in sensing proximity to the magnet 8100 and therefore the magnet 8100 is not sensed by those Hall sensors.

In FIG. 18B, the arm 8102 and magnet 8100 are in a second position as a result of the electrosurgical instrument being placed in an energy connection state (i.e., the energy transmission cable is received in the connector receptacle as in the embodiment of FIG. 17B). In this position, the magnet 8102 is in sensing proximity to both of the Hall sensors 8120A, 8120B, as shown by the region S, which indicates the magnetic flux range of the magnet 8100 having a strength sufficient to be detected by the Hall sensors 8120A, 8120B. In the second position, again as depicted by the region S, the Hall sensors 8118A, 8118B are not in sensing proximity to the magnet 8100 and therefore the magnet 8100 is not sensed by those Hall sensors.

Turning now to FIG. 18C, the schematic illustration depicts an installed position of the electrosurgical instrument housing 8104 relative to the actuation interface assembly 8114, however the energy connection state is ambiguous as a result of the energy transmission cable being received in the connector receptacle to a degree sufficient to move the arm 8102 but not completely engaged such that the arm 8102 moves to the second position of FIG. 18B. In this configuration wherein the arm 8102 is either in a state of travel between or otherwise placed in a position between the first and second positions shown in FIGS. 18A and 18B, respectively, the magnet 8100 is in sensing proximity of one Hall sensor of each of the Hall sensor pairs. That is, as shown by the region S, when the magnet 8100 is located between the first and second positions, it is in sensing proximity to Hall sensor 8118B and 8120B. In this position and based on the Hall sensor 8118B and 8120B sensing the magnet 8100 and sending signals to the controller 3004, the controller can determine that the electrosurgical instrument is in an installed position but that the energy connection state is ambiguous. In this case, the controller can control the system to prohibit energy delivery from an energy generator to the electrosurgical instrument, and optionally also provide a fault detection feedback signal, for example, to provide visual and/or audible feedback indicating that the energy connection state is ambiguous and/or the cable connection should be verified.

Providing the Hall sensor and magnet configuration of the exemplary embodiment of FIGS. 18A-18C can ensure that if the instrument is in an installed position, the magnet is always detected regardless of the energy connection state of the electrosurgical instrument and regardless of the position of the magnet throughout its travel between the first position and the second position. In this manner, there is a reduced risk of mistakenly determining that the electrosurgical instrument is not installed when it actually is and is in a state of potential energy connection (e.g., the energy transmission cable is plugged in sufficiently for electrical contact to be made in the instrument housing). Such an incorrect determination could lead to an ambiguity if an energy input command is provided at the surgeon side console, thereby resulting in potential inadvertent energy activation at the electrosurgical instrument.

As those of ordinary skill in the art will appreciate, a variety of mechanisms may be utilized to provide signals to a controller of a teleoperated surgical system to determine that an instrument is in an installed position at an actuation interface assembly of a manipulator arm and/or the energy connection state of an installed electrosurgical instrument. For example, those having ordinary skill in the art will appreciate that the exemplary embodiments shown and described with respect to FIGS. 17A-18C can be modified to incorporate various types of sensing technologies that can be implemented to alter a state in reaction to the energy connection state of the electrosurgical instrument (e.g., in response to the energy transmission cable being in a connected or unconnected state), including but not limited to, for example, optical, magnetic, electrical (e.g., switches), etc.

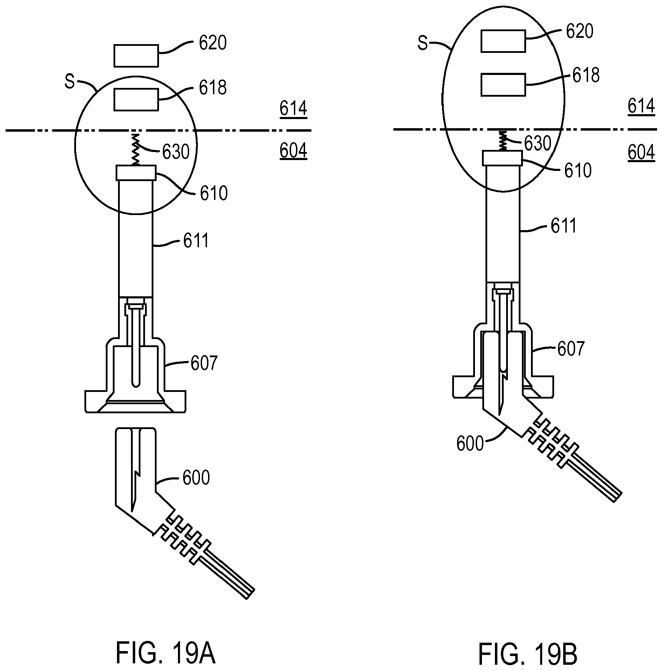

FIGS. 19A-24 depict some exemplary, nonlimiting alternative embodiments that are contemplated by the present disclosure. With reference to FIG. 19A and 19B, a schematic view of a set of Hall sensors 618 and 620 associated with the actuation interface assembly 614 and a magnet 610 on an arm 611 in the electrosurgical instrument housing 604 that moves up and down in response to an energy transmission cable 600 being placed in engagement with the housing is depicted. FIG. 19A shows the electrosurgical instrument not in an energy connection state (e.g., energy transmission cable 600 not received in the connector receptacle 607) and FIG. 19B shows the electrosurgical instrument in an energy connection state (e.g., energy transmission cable 600 received in the connector receptacle 607 and in electrical connection with electrical contact 609). In FIG. 19A, the magnet 610 on the proximal end of the arm 611 is in sensing proximity S to the Hall sensor 618, which can provide a signal to a controller at the control cart that the electrosurgical instrument is in an installed position. In FIG. 19B, the connector cable 600 acts on the arm 611 to move the arm 611 and the magnet 610 upward, thereby positioning the magnet 610 in sensing proximity, designated by S, of both Hall sensors 618 and 620. This provides another signal to the controller of the system that the electrosurgical instrument is in an energy connection state. A spring return 630 can be used to ensure the arm 611 returns to the position of FIG. 19A upon removal of the cable 600.

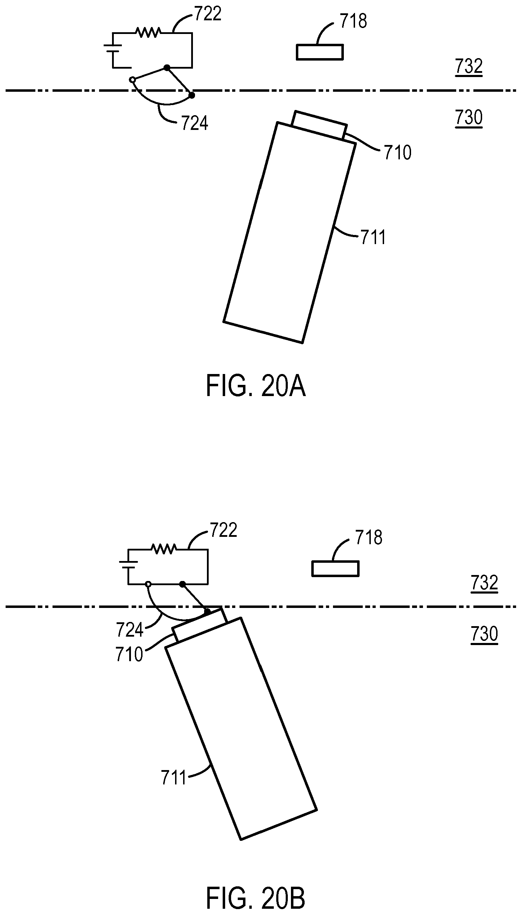

In another exemplary embodiment, as opposed to moving into the range of a second one or pair of Hall sensors, movement of the arm in response to the engagement of the energy transmission cable could cause the arm to act on a mechanical switch, for example through a sterile adaptor of an actuation interface assembly, to close a circuit in the actuation interface assembly. FIGS. 20A and 20B show a schematic depiction of an exemplary embodiment showing such a configuration using an arm 711 in an electrosurgical instrument housing 730 similar to that of the embodiment of FIGS. 17A and 17B. Such a mechanical switch embodiment can be configured for use with the embodiments of FIGS. 17A, 17B, 19A, and 19B, for example, and a magnet 710 on the arm 711 can be sensed by one or more Hall sensors 718, which are associated with an actuation interface assembly 732, in the first position (e.g., not in energy connection state; FIG. 20A) to provide the signal to the controller indicating the instrument is installed. Then, upon movement of the arm to the second position (e.g., energy connection state; FIG. 20B) as a result of the connection of the energy transmission cable, the arm 711 can engage a cam feature 724 to mechanically close a switch on a circuit 722 to send the signal to the controller indicating the energy connection state. In an alternative embodiment, the switch feature of FIG. 20A and 20B may be provided through an optical sensing mechanism, for example, a portion of the cam feature could move into and out of an interrupting position of a light path, which in turn could send a signal to the controller or otherwise close circuit. Those having ordinary skill in the art would understand how to modify the sensor configuration to implement such an optical sensing scheme.

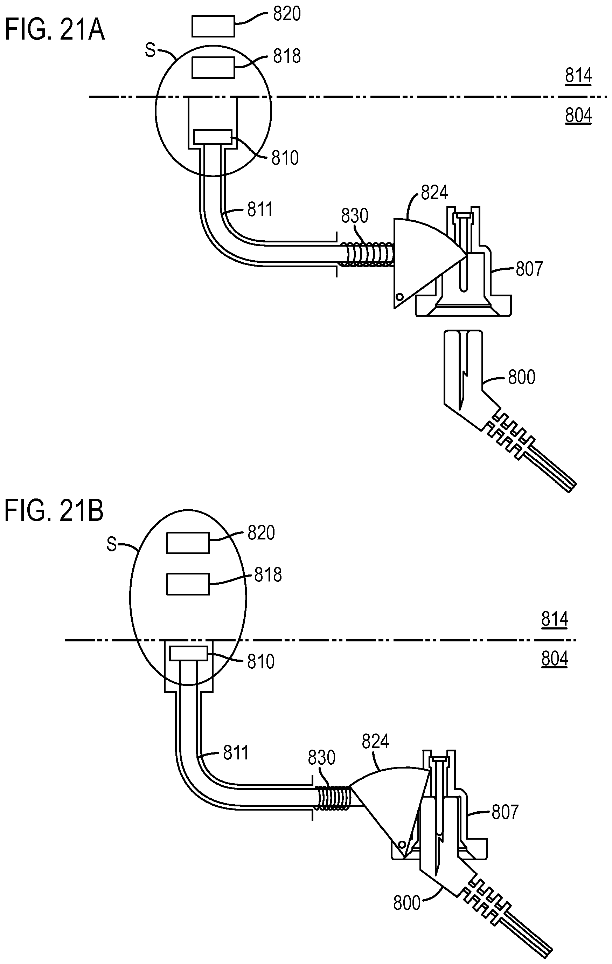

FIGS. 21A and 21B schematically depict an embodiment similar to FIGS. 19A and 19B except that an arm 811 carrying a magnet 810 on its proximal end is in connection with a spring-biased (e.g., via spring 830) rotary cam 824 that interacts with the energy transmission cable 800 to move the arm 811 and magnet 810 disposed on the proximal end of the arm 811 up and down in the electrosurgical instrument housing 804 and from sensing proximity of only one Hall sensor 818 (see region S in FIG. 21 A) to sensing proximity of two Hall sensors 818 and 820 (see region S in FIG. 21B). Hall sensors 818 and 820 may be associated with an actuation interface assembly 814. Thus, when the energy transmission cable 800 is not received in the receptacle housing 807, the magnet 810 is in the position shown in FIG. 21A and the surgical instrument is determined to not be in an energy connection state. When the energy transmission cable 800 is received in the receptacle housing 807, the magnet is moved to the position shown in FIG. 21B and the surgical instrument is determined to be in an energy connection state.

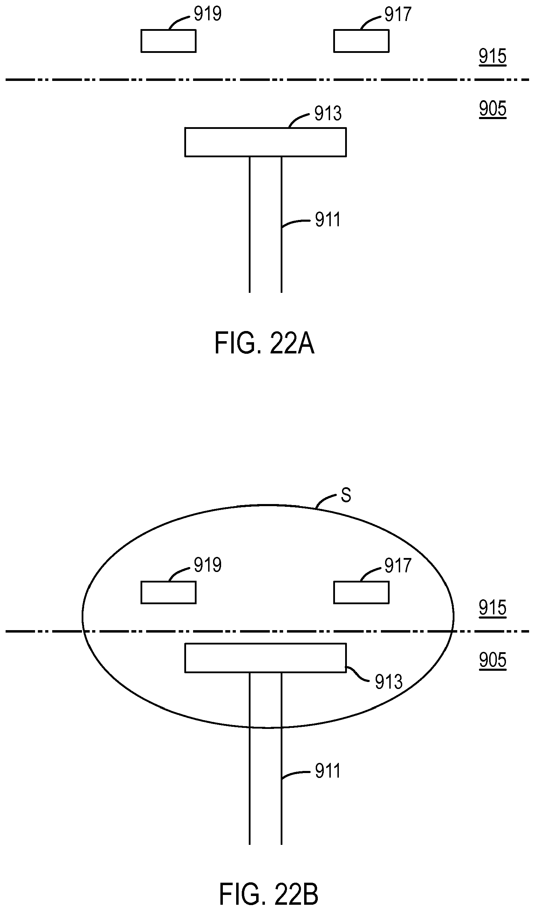

In various exemplary embodiments, a permanent magnet and Hall sensor could be used in combination as the sensing mechanism in the actuation interface assembly, and a steel or otherwise magnetizable member could be used in the electrosurgical instrument housing. With reference to the exemplary embodiment depicted schematically in FIGS. 22A and 22B, a magnetizable member 913 is provided within the electrosurgical housing 905. The magnetizable member 913 can be provided on an arm 911 that is movable from the position shown in FIG. 22A to the position shown in FIG. 22B, for example, in response to the energy transmission cable (not shown) being disengaged or engaged with the housing 905, as has been described above with respect to other exemplary embodiments. In the position of FIG. 22A, the member 913 is not in proximity to a permanent magnet 919 and Hall sensor 917 such that the member 913 is nonmagnetized and is accordingly not sensed by the Hall sensor 917. Permanent magnet 919 and Hall sensor 917 may be associated with an actuation interface assembly 915, according to an exemplary embodiment. In FIG. 22B, showing an energy connection state of the surgical instrument, the position of the member 913 is such that it is within the range of the permanent magnet 919 and the Hall sensor 917 such that the permanent magnet 919 causes the member 913 to become magnetized. The magnetic flux range of the member 913 in the position of FIG. 22B is in sensing proximity, as indicated by the region S, to the Hall sensor 917, which thereby can provide a signal to the controller to indicate an electrosurgical instrument is in an energy connection state.

In yet another exemplary embodiment, it is contemplated that a readable and writable memory device, such as, for example, an RFID tag and/or a memory chip, provided on the electrosurgical instrument could be configurable so as to change information upon engagement of the energy transmission cable to the electrosurgical instrument. For example, the readable and writable memory device (e.g., RFID tag and/or memory chip) could be configurable such that in addition to providing instrument information, including the type (e.g., energy type) of the instrument, the information stored could be changed from indicating a no energy connection state to an energy connection state depending on whether or not the energy transmission cable is in mating electrical connection with the housing.

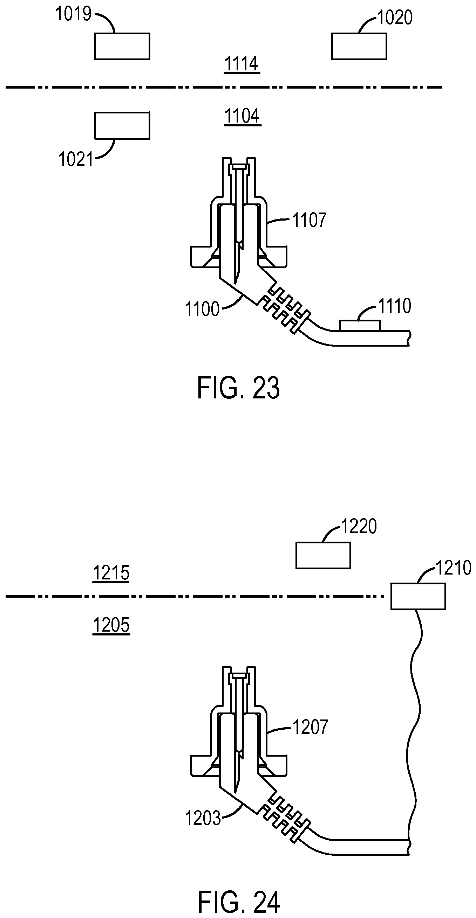

In various alternative exemplary embodiments, the present disclosure contemplates sensing the cable presence by providing one or more sensing mechanisms on the cable itself. For example, with reference to FIG. 23, it may be possible to, in addition to a first RFID tag 1021 associated with the electrosurgical instrument (e.g., disposed at housing 1104), an additional RFID tag 1110 can be placed on the energy transmission cable 1100 (which is shown coupled to electrosurgical instrument connector receptacle 1107). More than one RFID reader (e.g., two readers 1019 and 1020 shown in FIG. 10) could be provided at an actuation interface assembly 1114 and both the electrosurgical instrument and the first RFID tag 1021 and RFID tag 1110 may be readable only by a respective one of the RFID readers, for example, based on proximity to the same. For example, in the exemplary embodiment of FIG. 23, RFID reader 1019 can sense RFID tag 1021 when the electrosurgical instrument is in an installed position relative to the actuation interface assembly 1114, and RFID reader 1020 could sense RFID tag 1110 when the energy transmission cable 1200 is in mating engagement with the electrosurgical instrument connector receptacle 1107 in the housing 1104. Placement of the RFID tag and reader associated with reading the energy transmission cable could be selected so that the RFID tag on the cable comes into sensing proximity to the reader when the cable is placed in mating electrical connection with the electrosurgical instrument.

In accordance with an alternative exemplary embodiment, with reference to FIG. 24, a magnet 1210 or other sensing mechanism may be provided on the energy transmission cable 1203. A magnet 1210 or other sensing mechanism may be attached to the cable 1203 by a leash and positionable by a user such that when the cable is placed in mating connection with the connector receptacle 1207 of the electrosurgical instrument housing 1205, the magnet 1210 or other sensing mechanism is able to be positioned relative to an actuation interface assembly 1215 so as to be placed in sensing proximity with a sensor 1220, which may be for example a Hall sensor 1220. To facilitate accurate placement, the sensor 1220 may be positioned external to the actuation interface assembly 1215 and/or if placed internally, some designation could be provided on the actuation interface assembly 1215 observable by a user so as to facilitate placement of the sensing mechanism.

In yet another exemplary embodiment, the energy transmission cable may be directly sensed at the patient side cart by, for example, interrupting a light path or other optical sensing mechanism that changes state from a disconnected configuration of the cable to a mating electrical connection configuration of the cable with the electrosurgical instrument. As another example, when the transmission cable makes electrical contact with the electrosurgical instrument, a light circuit could be closed to light up an LED or other light that is in turn sensed by a sensor in the actuation interface assembly or otherwise at the patient side cart.

In another exemplary embodiment, a method of supplying flux includes receiving first data and second data at a control system. The first data may identify which of a plurality of remotely-controllable kinematic flux delivery structures is operationally coupled to which of a plurality of flux supply pathways. The second data may identify which of the remotely-controllable kinematic flux delivery structures is operationally coupled to which of a plurality of kinematic support structures. The method may further include, in response to an input command signal to deliver flux from a selected one of the remotely-controllable kinematic flux delivery structures that is received by the control system, sending a signal to supply flux from the flux supply pathway operationally coupled to the selected one of the remotely-controllable kinematic flux delivery structures, based on the first data and the second data.

Exemplary embodiments, including the various operational methods described herein, can be implemented in computing hardware (computing apparatus) and/or software, such as (in a non-limiting example) any computer that can store, retrieve, process and/or output data and/or communicate with other computers. The results produced can be displayed on a display of the computing hardware. One or more programs/software comprising algorithms to affect the various responses and signal processing in accordance with various exemplary embodiments of the present disclosure can be implemented by a processor, such as data interface module, of or in conjunction with the control cart including core processor and may be recorded on computer-readable media including computer-readable recording and/or storage media. Examples of the computer-readable recording media include a magnetic recording apparatus, an optical disk, a magneto-optical disk, and/or a semiconductor memory (for example, RAM, ROM, etc.). Examples of the magnetic recording apparatus include a hard disk device (HDD), a flexible disk (FD), and a magnetic tape (MT). Examples of the optical disk include a DVD (Digital Versatile Disc), a DVD-RAM, a CD-ROM (Compact Disc - Read Only Memory), and a CD-R (Recordable)/RW.

Further modifications and alternative embodiments will be apparent to those of ordinary skill in the art in view of the disclosure herein. For example, the systems and the methods may include additional components or steps that were omitted from the diagrams and description for clarity of operation. Accordingly, this description is to be construed as illustrative only and is for the purpose of teaching those skilled in the art the general manner of carrying out the present teachings. It is to be understood that the various embodiments shown and described herein are to be taken as exemplary. Elements and materials, and arrangements of those elements and materials, may be substituted for those illustrated and described herein, parts and processes may be reversed, and certain features of the present teachings may be utilized independently, all as would be apparent to one skilled in the art after having the benefit of the description herein. Changes may be made in the elements described herein without departing from the scope of the present teachings and following claims. In particular, based on the present disclosure, those of ordinary skill in the art would understand a variety of differing sensing mechanisms and arrangements that could be employed to detect the presence of various types of flux transmission conduits in a connection state with a surgical instrument, and the particular sensing mechanisms and arrangements set forth in the illustrated embodiments and described above should not be construed as limiting. For example, the depicted energy transmission cables could have differing connection features, or hoses with luer fittings or other connection features to connect to the surgical instrument can be used, with those of ordinary skill in the art appreciating that various modifications to the interaction between such flux transmission conduits with the surgical instrument housing to carry out the teachings and principles of the present disclosure.

It is to be understood that the particular examples and embodiments set forth herein are nonlimiting, and modifications to structure, dimensions, materials, and methodologies may be made without departing from the scope of the present teachings.

Other embodiments in accordance with the present disclosure will be apparent to those skilled in the art from consideration of the specification and practice of the invention disclosed herein. It is intended that the specification and examples be considered as exemplary only, with a true scope being indicated by the following claims.

## Claims

1. An electrosurgical energy delivery system, comprising:
an electrosurgical instrument (1202, 1302) comprising:
an electronic circuit (280) encoded with first identification data corresponding to the electrosurgical instrument (1202, 1302);
a data storage device (2252) configured to store second identification data corresponding to the electrosurgical instrument (1202, 1302), the data storage device being configured to transmit a second data signal including the second identification data; and
an instrument connector interface (1200, 1300), the instrument connector interface (1200, 1300) comprising:
an instrument data signal transmission connector feature (1206, 1306) in communication with the electronic circuit (280) to transmit a first data signal including the first identification data, and
an instrument electrosurgical energy transmission connector feature (1204, 1304) that provides an electrosurgical energy pathway for the electrosurgical instrument (1202, 1302); and
an electrosurgical energy transmission conduit comprising:
a conduit data signal transmission pathway,
a conduit electrosurgical energy transmission pathway, and
a conduit connector interface (910, 920, 1010, 1020) comprising:
a conduit data signal transmission connector feature (914, 924, 1016a, 1016b, 1026) in communication with the conduit data signal transmission pathway, and
a pair of conduit electrosurgical energy transmission connector features (912, 922, 1012,1014, 1022, 1024) in communication with the conduit electrosurgical energy transmission pathway, the pair of conduit electrosurgical transmission connector features (912, 922, 1012,1014, 1022, 1024) being separate from the conduit data signal transmission connector feature (914, 924, 1016a, 1016b, 1026),
wherein the instrument connector interface (1200, 1300) and the conduit connector interface (910, 920, 1010, 1020) are configured to mate with one another to place the electrosurgical instrument (1202, 1302) and the electrosurgical energy transmission conduit in data signal transmission communication and electrosurgical energy transmission communication.

2. The system of claim 1, wherein the pair of conduit electrosurgical energy transmission connector features (912, 922, 1012,1014, 1022, 1024) are spaced from and aligned with each other, the conduit electrosurgical energy transmission connector features (912, 922, 1012,1014, 1022, 1024) being chosen from female features and male features.

3. The system of claim 1, wherein the conduit data signal transmission connector feature (914, 924, 1016a, 1016b, 1026) of the conduit connector interface (910, 920, 1010, 1020) of the electrosurgical energy transmission conduit comprises a pair of conduit data signal transmission connector features spaced from the pair of conduit electrosurgical energy transmission connector features (912, 922, 1012, 1014, 1022, 1024).

4. The system of claim 3, wherein the pair of conduit data signal transmission connector features comprise female terminals.

5. The system of claim 1, wherein the pair of conduit electrosurgical energy transmission connector features (912, 922, 1012, 1014, 1022, 1024) are female connector features.

6. The system of claim 1, wherein the conduit electrosurgical energy transmission pathway is chosen from a monopolar conduit electrosurgical energy transmission pathway and a bipolar conduit electrosurgical energy transmission pathway, wherein the one of the pair of conduit electrosurgical energy transmission connector features (1014, 1024) of the monopolar conduit electrosurgical energy transmission pathway is sized smaller than the other of the pair of conduit electrosurgical energy transmission connector feature (1012, 1022) of the monopolar conduit electrosurgical energy transmission pathway.

7. The system of claim 6, wherein the conduit connector interface (910, 920, 1010, 1020) is chosen from a monopolar conduit electrosurgical energy connector interface and a bipolar conduit electrosurgical energy connector interface.

8. The system of claim 1, wherein the conduit connector interface (910, 920, 1010, 1020) is further configured to mate with another instrument connector interface having another instrument electrosurgical energy transmission connector feature and without having another instrument data signal transmission connector feature.

9. The system of claim 1, wherein the electrosurgical energy transmission conduit further comprises an additional conduit connector interface disposed at an opposite end of the electrosurgical energy transmission conduit from the conduit connector interface (910, 920, 1010, 1020), the additional conduit connector interface comprising:
an additional conduit data signal transmission connector feature in communication with the conduit data signal transmission pathway;
an additional pair of conduit electrosurgical energy transmission connector features in communication with the conduit electrosurgical energy transmission pathway, the additional conduit connector interface configured to mate with an electrosurgical energy supply source connector interface having a supply source electrosurgical energy transmission connector feature and a supply source data signal transmission connector feature; and
wherein the additional conduit connector interface is further configured to mate with another second electrosurgical energy supply source connector interface having another supply source electrosurgical energy transmission connector feature and without having another supply source data signal transmission connector feature.

10. The system of claim 1, further comprising:
a processor (200, 2200, 3200, 4200) configured to receive the first identification data relating to the electrosurgical instrument (1202, 1302) when the electrosurgical instrument (1202, 1302) is operationally coupled with an electrosurgical energy supply source, and receive the second identification data relating to the electrosurgical instrument (1202, 1302) when the electrosurgical instrument (1202, 1302) is operationally coupled with a support structure.

11. The system of claim 10, wherein the support structure (2140a-d) comprises:
a data reader (2254) configured to obtain the second identification data relating to the electrosurgical instrument (1202, 1302) from the data storage device (2252) when the electrosurgical instrument (1202, 1302) is operationally coupled with the support structure, the data reader configured to transmit the second identification data to the processor.

12. The system of claim 11, wherein the data storage device provided at the electrosurgical instrument (1202, 1302) is a radio frequency identification (RFID) tag, and the data reader provided at the support structure is an RFID reader, and wherein the RFID reader reads the second identification data from the RFID tag.

13. The system of claim 10, wherein the processor is further configured to identify, based on the first identification data and the second identification data, that the electrosurgical instrument (1202, 1302) is operationally coupled with an electrosurgical energy supply source and that the electrosurgical instrument (1202, 1302) is also operationally coupled with the support structure.

14. The system of claim 13, wherein the processor is further configured to send a signal to the electrosurgical energy supply source (2206) to supply energy through the conduit electrosurgical energy transmission pathway to the electrosurgical instrument (1202, 1302) upon identifying that the electrosurgical instrument (1202, 1302) is operationally coupled with the support structure and operationally coupled with the electrosurgical energy supply source.

## Patentansprüche

1. Ein elektrochirurgisches Energiezufuhrsystem, das Folgendes beinhaltet:
ein elektrochirurgisches Instrument (1202, 1302), das Folgendes beinhaltet:
eine elektronische Schaltung (280), die mit ersten Identifikationsdaten kodiert ist, die dem elektrochirurgischen Instrument (1202, 1302) entsprechen;
eine Datenspeichervorrichtung (2252), die dazu ausgelegt ist, zweite Identifikationsdaten zu speichern, die dem elektrochirurgischen Instrument (1202, 1302) entsprechen, wobei die Datenspeichervorrichtung dazu ausgelegt ist, ein zweites Datensignal zu übertragen, das die zweiten Identifikationsdaten umfasst; und
eine Instrumentenverbindungsschnittstelle (1200, 1300), wobei die Instrumentenverbindungsschnittstelle (1200, 1300) Folgendes beinhaltet:
ein Instrumentendatensignalübertragungs-Verbindungsmerkmal (1206, 1306) in Kommunikation mit der elektronischen Schaltung (280), um ein erstes Datensignal zu übertragen, das die ersten Identifikationsdaten umfasst, und
ein elektrochirurgisches Instrument-Energieübertragungsverbindungsmerkmal (1204, 1304), das eine elektrochirurgische Energiebahn für das elektrochirurgische Instrument (1202, 1302) bereitstellt; und
eine elektrochirurgische Energieübertragungsleitung die Folgendes beinhaltet:
eine Leitungs-Datensignalübertragungsbahn,
eine elektrochirurgische Leitungs-Energieübertragungsbahn, und
eine Leitungsverbindungsschnittstelle (910, 920, 1010, 1020), die Folgendes beinhaltet:
ein Leitungs-Datensignalübertragungsverbindungsmerkmal (914, 924, 1016a, 1016b, 1026) in Kommunikation mit der Leitungs-Datensignalübertragungsbahn, und
ein Paar von elektrochirurgischen Leitungs-Energieübertragungsverbindungsmerkmalen (912, 922, 1012, 1014, 1022, 1024) in Kommunikation mit der elektrochirurgischen Leitungs-Energieübertragungsbahn, wobei das Paar von elektrochirurgischen Leitungs-Übertragungsverbindungsmerkmalen (912, 922, 1012, 1014, 1022, 1024) separat von dem Leitungs-Datensignalübertragungsverbindungsmerkmal (914, 924, 1016a, 1016b, 1026) ist,
wobei die Instrumentenverbindungsschnittstelle (1200, 1300) und die Leitungsverbindungsschnittstelle (910, 920, 1010, 1020) dazu ausgelegt sind, miteinander zusammenzupassen, um das elektrochirurgische Instrument (1202, 1302) und die elektrochirurgische Energieübertragungsleitung in Datensignalübertragungskommunikation und elektrochirurgische Energieübertragungskommunikation miteinander zu bringen.

2. System nach Anspruch 1, wobei das Paar von elektrochirurgischen Leitungs-Energieübertragungsverbindungsmerkmalen (912, 922, 1012, 1014, 1022, 1024) zueinander beabstandet und miteinander ausgerichtet ist, wobei die elektrochirurgischen Leitungs-Energieübertragungsverbindungsmerkmale (912, 922, 1012,1014, 1022, 1024) aus Aufnahmemerkmalen und Steckmerkmalen ausgewählt sind.

3. System nach Anspruch 1, wobei das Leitungs-Datensignalübertragungsverbindungsmerkmal (914, 924, 1016a, 1016b, 1026) der Leitungsverbindungsschnittstelle (910, 920, 1010, 1020) der elektrochirurgischen Energieübertragungsleitung ein Paar von Leitungs-Datensignalübertragungsverbindungsmerkmalen beinhaltet, das zu dem Paar von elektrochirurgischen Leitungs-Energieübertragungsverbindungsmerkmalen (912, 922, 1012, 1014, 1022, 1024) beabstandet ist.

4. System nach Anspruch 3, wobei das Paar von Leitungs-Datensignalübertragungsverbindungsmerkmalen Aufnahmeanschlüsse beinhaltet.

5. System nach Anspruch 1, wobei das Paar von elektrochirurgischen Leitungs-Energieübertragungsverbindungsmerkmalen (912, 922, 1012, 1014, 1022, 1024) Aufnahmeverbindungsmerkmale sind.

6. System nach Anspruch 1, wobei die elektrochirurgische Leitungs-Energieübertragungsbahn aus einer monopolaren elektrochirurgischen Leitungs-Energieübertragungsbahn und einer bipolaren elektrochirurgischen Leitungs-Energieübertragungsbahn ausgewählt ist, wobei das eine von dem Paar von elektrochirurgischen Leitungs-Energieübertragungsverbindungsmerkmalen (1014, 1024) der monopolaren elektrochirurgischen Leitungs-Energieübertragungsbahn kleiner dimensioniert ist als das andere von dem Paar von elektrochirurgischen Leitungs-Energieübertragungsverbindungsmerkmalen (1012, 1022) der monopolaren elektrochirurgischen Leitungs-Energieübertragungsbahn.

7. System nach Anspruch 6, wobei die Leitungsverbindungsschnittstelle (910, 920, 1010, 1020) aus einer monopolaren elektrochirurgischen Leitungs-Energieverbindungsschnittstelle und einer bipolaren elektrochirurgischen Leitungs-Energieverbindungsschnittstelle ausgewählt ist.

8. System nach Anspruch 1, wobei die Leitungsverbindungsschnittstelle (910, 920, 1010, 1020) weiter dazu ausgelegt ist, mit einer anderen Instrumentenverbindungsschnittstelle zusammenzupassen, die ein anderes elektrochirurgisches Instrument-Energieübertragungsverbindungsmerkmal aufweist und kein anderes Instrument-Datensignalübertragungsverbindungsmerkmal aufweist.

9. System nach Anspruch 1, wobei die elektrochirurgische Energieübertragungsleitung ferner eine zusätzliche Leitungsverbindungsschnittstelle beinhaltet, die an einem der Leitungsverbindungsschnittstelle (910, 920, 1010, 1020) gegenüberliegenden Ende der elektrochirurgischen Energieübertragungsleitung angeordnet ist, wobei die zusätzliche Leitungsverbindungsschnittstelle Folgendes beinhaltet:
ein zusätzliches Leitungs-Datensignalübertragungsverbindungsmerkmal in Kommunikation mit der Leitungs-Datensignalübertragungsbahn;
ein zusätzliches Paar von elektrochirurgischen Leitungs-Energieübertragungsverbindungsmerkmalen in Kommunikation mit der elektrochirurgischen Leitungs-Energieübertragungsbahn, wobei die zusätzliche Leitungsverbindungsschnittstelle dazu ausgelegt ist, mit einer elektrochirurgischen Energieversorgungsquellen-Verbindungsschnittstelle zusammenzupassen, die ein elektrochirurgisches Energieübertragungsverbindungsmerkmal für die Versorgungsquelle und ein Datensignalübertragungsverbindungsmerkmal für die Versorgungsquelle aufweist; und
wobei die zusätzliche Leitungsverbindungsschnittstelle ferner dazu ausgelegt ist, mit einer anderen zweiten elektrochirurgischen Energieversorgungsquellen-Verbindungsschnittstelle zusammenzupassen, die ein anderes elektrochirurgisches Energieübertragungsverbindungsmerkmal für die Versorgungsquelle aufweist und kein anderes Datensignalübertragungsverbindungsmerkmal für die Versorgungsquelle aufweist.

10. System nach Anspruch 1, das ferner Folgendes beinhaltet:
einen Prozessor (200, 2200, 3200, 4200), der dazu ausgelegt ist, die ersten Identifikationsdaten zu empfangen, die sich auf das elektrochirurgische Instrument (1202, 1302) beziehen, wenn das elektrochirurgische Instrument (1202, 1302) betriebsfähig mit einer elektrochirurgischen Energieversorgungsquelle gekoppelt ist, und die zweiten Identifikationsdaten zu empfangen, die sich auf das elektrochirurgische Instrument (1202, 1302) beziehen, wenn das elektrochirurgische Instrument (1202, 1302) betriebsfähig mit einer Unterstützungsstruktur gekoppelt ist.

11. System nach Anspruch 10, wobei die Unterstützungsstruktur (2140a-d) Folgendes beinhaltet:
ein Datenlesegerät (2254), das dazu ausgelegt ist, die zweiten Identifikationsdaten, die sich auf das elektrochirurgische Instrument (1202, 1302) beziehen, von der Datenspeichervorrichtung (2252) zu erhalten, wenn das elektrochirurgische Instrument (1202, 1302) betriebsfähig mit der Unterstützungsstruktur gekoppelt ist, wobei das Datenlesegerät dazu ausgelegt ist, die zweiten Identifikationsdaten zu dem Prozessor zu übertragen.

12. System nach Anspruch 11, wobei die an dem elektrochirurgischen Instrument (1202, 1302) bereitgestellte Datenspeichervorrichtung eine Hochfrequenzidentifikations(RFID)-Markierung ist, und das an der Unterstützungsstruktur bereitgestellte Datenlesegerät ein RFID-Lesegerät ist, und wobei das RFID-Lesegerät die zweiten Identifikationsdaten von der RFID-Markierung liest.

13. System nach Anspruch 10, wobei der Prozessor ferner dazu ausgelegt ist, basierend auf den ersten Identifikationsdaten und den zweiten Identifikationsdaten festzustellen, dass das elektrochirurgische Instrument (1202, 1302) betriebsfähig mit einer elektrochirurgischen Energieversorgungsquelle gekoppelt ist und dass das elektrochirurgische Instrument (1202, 1302) auch betriebsfähig mit der Unterstützungsstruktur gekoppelt ist.

14. System nach Anspruch 13, wobei der Prozessor ferner dazu ausgelegt ist, ein Signal an die elektrochirurgische Energieversorgungsquelle (2206) zu senden, um dem elektrochirurgischen Instrument (1202, 1302) Energie durch die elektrochirurgische Leitungs-Energieübertragungsbahn zu liefern, nachdem festgestellt worden ist, dass das elektrochirurgische Instrument (1202, 1302) betriebsfähig mit der Unterstützungsstruktur gekoppelt ist und betriebsfähig mit der elektrochirurgischen Energieversorgungsquelle gekoppelt ist.

## Revendications

1. Système de délivrance d'énergie électrochirurgicale, comprenant :
un instrument électrochirurgical (1202, 1302) comprenant :
un circuit électronique (280) codé avec une première donnée d'identification correspondant à l'instrument chirurgical (1202, 1302) ;
un dispositif de stockage de données (2252) configuré pour stocker une deuxième donnée d'identification correspondant à l'instrument électrochirurgical (1202, 1302), le dispositif de stockage de données étant configuré pour transmettre un deuxième signal de donnée incluant la deuxième donnée d'identification ; et
une interface de connecteur d'instrument (1200, 1300), l'interface de connecteur d'instrument (1200, 1300) comprenant :
un élément connecteur de transmission de signal de donnée d'instrument (1206, 1306) en communication avec le circuit électronique (280) pour transmettre un premier signal de donnée incluant la première donnée d'identification, et
un élément connecteur de transmission d'énergie électrochirurgicale d'instrument (1204, 1304) qui fournit une voie de passage d'énergie électrochirurgicale pour l'instrument électrochirurgical (1202, 1302) ; et
un conduit de transmission d'énergie électrochirurgicale comprenant :
une voie de passage de transmission de signal de donnée de conduit,
une voie de passage de transmission d'énergie électrochirurgicale de conduit, et
une interface de connecteur de conduit (910, 920, 1010, 1020) comprenant :
un élément connecteur de transmission de signal de donnée de conduit (914, 924, 1016a, 1016b, 1026) en communication avec la voie de passage de transmission de signal de donnée de conduit, et
une paire d'éléments connecteurs de transmission d'énergie électrochirurgicale de conduit (912, 922, 1012, 1014, 1022, 1024) en communication avec la voie de passage de transmission d'énergie électrochirurgicale de conduit, la paire d'éléments connecteurs de transmission de conduit (912, 922, 1012, 1014, 1022, 1024) étant séparée de l'élément connecteur de transmission de signal de donnée de conduit (914, 924, 1016a, 1016b, 1026),
dans lequel l'interface de connecteur d'instrument (1200, 1300) et l'interface de connecteur de conduit (910, 920, 1010, 1020) sont configurées pour s'accoupler l'une avec l'autre pour placer l'instrument électrochirurgical (1202, 1302) et le conduit de transmission d'énergie électrochirurgicale en communication de transmission de signal de donnée et en communication de transmission d'énergie électrochirurgicale.

2. Système selon la revendication 1, dans lequel la paire d'éléments connecteurs de transmission d'énergie électrochirurgicale de conduit (912, 922, 1012, 1014, 1022, 1024) sont espacés l'un de l'autre et alignés l'un avec l'autre, les éléments connecteurs de transmission d'énergie électrochirurgicale de conduit (912, 922, 1012, 1014, 1022, 1024) étant choisis parmi des éléments femelles et des éléments mâles.

3. Système selon la revendication 1, dans lequel l'élément connecteur de transmission de signal de donnée de conduit (914, 924, 1016a, 1016b, 1026) de l'interface de connecteur de conduit (910, 920, 1010, 1020) du conduit de transmission d'énergie électrochirurgicale comprend une paire d'éléments connecteurs de transmission de signal de donnée de conduit espacée de la paire d'éléments de connecteur de transmission d'énergie électrochirurgicale de conduit (912, 922, 1012, 1014, 1022, 1024).

4. Système selon la revendication 3, dans lequel la paire d'éléments connecteurs de transmission de signal de donnée de conduit comprend des terminaux femelles.

5. Système selon la revendication 1, dans lequel la paire d'éléments connecteurs de transmission d'énergie électrochirurgicale de conduit (912, 922, 1012, 1014, 1022, 1024) sont des éléments connecteurs femelles.

6. Système selon la revendication 1, dans lequel la voie de passage de transmission d'énergie électrochirurgicale de conduit est choisie parmi une voie de passage de transmission d'énergie électrochirurgicale de conduit monopolaire et une voie de passage de transmission d'énergie électrochirurgicale de conduit bipolaire, dans lequel l'un de la paire d'éléments connecteurs de transmission d'énergie électrochirurgicale de conduit (1014, 1024) de la voie de passage de transmission d'énergie électrochirurgicale de conduit monopolaire a une plus petite dimension que l'autre de la paire d'éléments connecteurs de transmission d'énergie électrochirurgicale de conduit (1012, 1022) de la voie de passage de transmission d'énergie électrochirurgicale de conduit monopolaire.

7. Système selon la revendication 6, dans lequel l'interface de connecteur de conduit (910, 920, 1010, 1020) est choisie parmi une interface de connecteur d'énergie électrochirurgicale de conduit monopolaire et une interface de connecteur d'énergie électrochirurgicale de conduit bipolaire.

8. Système selon la revendication 1, dans lequel l'interface de connecteur de conduit (910, 920, 1010, 1020) est configurée en outre pour s'accoupler avec une autre interface de connecteur d'instrument ayant un autre élément connecteur de transmission d'énergie électrochirurgicale d'instrument et sans avoir un autre élément connecteur de transmission de signal de donnée d'instrument.

9. Système selon la revendication 1, dans lequel le conduit de transmission d'énergie électrochirurgicale comprend en outre une interface de connecteur de conduit supplémentaire disposée au niveau d'une extrémité opposée du conduit de transmission d'énergie électrochirurgicale à l'interface de connecteur de conduit (910, 920, 1010, 1020), l'interface de connecteur de conduit supplémentaire comprenant :
un élément connecteur de transmission de signal de donnée de conduit supplémentaire en communication avec la voie de passage de transmission de signal de donnée de conduit ;
une paire supplémentaire d'éléments connecteurs de transmission d'énergie électrochirurgicale de conduit en communication avec la voie de passage de transmission d'énergie électrochirurgicale de conduit,
l'interface de connecteur de conduit supplémentaire configurée pour s'accoupler avec une interface de connecteur de source d'alimentation en énergie électrochirurgicale ayant un élément connecteur de transmission d'énergie électrochirurgicale de source d'alimentation et un élément connecteur de transmission de signal de donnée de source d'alimentation ; et
dans lequel l'interface de connecteur de conduit supplémentaire est configurée en outre pour s'accoupler avec une autre deuxième interface de connecteur de source d'alimentation en énergie électrochirurgicale ayant un autre élément connecteur de transmission d'énergie électrochirurgicale de source d'alimentation et sans avoir un autre élément connecteur de transmission de signal de donnée de source d'alimentation.

10. Système selon la revendication 1, comprenant en outre :
un processeur (200, 2200, 3200, 4200) configuré pour recevoir la première donnée d'identification concernant l'instrument électrochirurgical (1202, 1302) quand l'instrument électrochirurgical (1202, 1302) est couplé de manière fonctionnelle avec une source d'alimentation en énergie électrochirurgicale, et recevoir la deuxième donnée d'identification concernant l'instrument électrochirurgical (1202, 1302) quand l'instrument électrochirurgical (1202, 1302) est couplé de manière fonctionnelle avec une structure de support.

11. Système selon la revendication 10, dans lequel la structure de support (2140a à d) comprend :
un lecteur de données (2254) configuré pour obtenir la deuxième donnée d'identification concernant l'instrument électrochirurgical (1202, 1302) à partir du dispositif de stockage de données (2252) quand l'instrument électrochirurgical (1202, 1302) est couplé de manière fonctionnelle avec la structure de support, le lecteur de données configuré pour transmettre la deuxième donnée d'identification au processeur.

12. Système selon la revendication 11, dans lequel le dispositif de stockage de données fourni au niveau de l'instrument électrochirurgical (1202, 1302) est une étiquette d'identification à radiofréquence (RFID), et le lecteur de données fourni au niveau de la structure de support est un lecteur RFID, et dans lequel le lecteur RFID lit la deuxième donnée d'identification à partir de l'étiquette RFID.

13. Système selon la revendication 10, dans lequel le processeur est configuré en outre pour identifier, sur la base de la première donnée d'identification et de la deuxième donnée d'identification, que l'instrument électrochirurgical (1202, 1302) est couplé de manière fonctionnelle avec une source d'alimentation en énergie électrochirurgicale et que l'instrument électrochirurgical (1202, 1302) est aussi couplé de manière fonctionnelle avec la structure de support.

14. Système selon la revendication 13, dans lequel le processeur est configuré en outre pour envoyer un signal à la source d'alimentation en énergie électrochirurgicale (2206) pour fournir de l'énergie à travers la voie de passage de transmission d'énergie électrochirurgicale de conduit à l'instrument électrochirurgical (1202, 1302) après identification que l'instrument électrochirurgical (1202, 1302) est couplé de manière fonctionnelle avec la structure de support et couplé de manière fonctionnelle avec la source d'alimentation en énergie électrochirurgicale.
